# FASCICULE DE BREVET EUROPEEN

(11) **EP 0 748 315 B1**
(45) Date de publication et mention de la délivrance du brevet: **07.01.2004**
(21) Numéro de dépôt: 95910603.0
(22) Date de dépôt: 27.02.1995
(51) Int. Cl.: C07D 233/84, C07D 233/68, C07D 233/90, A61K 31/415

(54) **UTILISATION DE DERIVES DE L'IMIDAZOLE AU TRAITEMENT D'AFFECTIONS IMPLIQUANT LES RECEPTEURS AT1 ET AT2 DE L'ANGIOTENSINE, CES PRODUITS, LEUR PREPARATION, LEUR APPLICATION A TITRE DE MEDICAMENTS ET LES COMPOSITIONS PHARMACEUTIQUES LES RENFERMANT**
VERWENDUNG VON IMIDAZOL-DERIVATEN ZUR BEHANDLUNG VON BESCHWERDEN AN DENEN ANGIOTENSIN REZEPTOREN AT1 UND AT2 BETEILIGT SIND, DIESE VERBINDUNGEN, DEREN HERSTELLUNG, VERWENDUNG ALS MEDIKAMENTE, UND PHARMAZEUTISCHE ZUSAMMENSETZUNGEN DIE SIE ENTHALTEN
USE OF IMIDAZOLE DERIVATIVES FOR TREATING DISORDERS INVOLVING ANGIOTENSIN RECEPTORS AT1 AND AT2, THESE PRODUCTS, PREPARATION THEREOF, USE THEREOF AS DRUGS, AND PHARMACEUTICAL COMPOSITIONS CONTAINING SAME

(30) Priorité: 04.03.1994 FR 9402518
(43) Date de publication de la demande: 18.12.1996
(60) Demande divisionnaire: 02006541.3
(73) Titulaire: Aventis Pharma S.A., 92160 Antony (FR)
(72) Inventeur: CORBIER, Alain, F-91370 Verrières-le-Buisson (FR); DEPREZ, Pierre, F-94320 Thiais (FR); FORTIN, Michel, F-75018 Paris (FR); GUILLAUME, Jacques, F-93190 Livry-Gargan (FR); HECKMANN, Bertrand, F-94230 Cachan (FR)
(86) Numéro de dépôt international: PCT/FR1995/000228
(87) Numéro de publication internationale: WO 1995/023792

(56) Documents cités:
- EP-A- 0 324 377
- EP-A- 0 409 332
- EP-A- 0 465 368
- EP-A- 0 479 479
- EP-A- 0 503 162
- EP-A- 0 505 098
- EP-A- 0 560 177
- EP-A- 0 577 023
- EP-A- 0 577 025
- WO-A-92/05784
- WO-A-94/28896
- CHEMICAL ABSTRACTS, vol. 120, no. 17, 25 Avril 1994, Columbus, Ohio, US; abstract no. 207919y, & BIOORG. MED. CHEM. LETT., vol.4, no.1, 1994 pages 69 - 74 E.M. NAYLOR AND AL. & CHEM. ABS. REGISTRY HANDBOOK SUPPL. 1994 (STN DATABASE)

## Description

La présente invention concerne une nouvelle utilisation de certains dérivés de l'imidazole au traitement d'affections impliquant les récepteurs AT₁ et AT₂ de l'Angiotensine, ces produits, leur préparation, leur application à titre de médicaments et les compositions pharmaceutiques les renfermant.

Le document EP-A-560177 décrit des dérivés de l'imidazole comme antagonistes de récepteur de l'Angiotensine II.

Les documents EP-A-577023 et EP-A-577025 décrivent des dérivés hétérocycliques comme antagonistes de récepteur de l'Angiotensine II.

Les documents EP-A-505098 et EP-A-479479 décrivent des dérivés de l'imidazole comme antagonistes de l'Angiotensine II.

Le document EP-A-324377 décrit des dérivés de l'imidazole comme antagonistes de récepteur de l'Angiotensine II.

L'Article Bioorg. Med. Chem. Lett. 1994, Vol.4(1), 69-74 décrit des dérivés de l'imidazole comme antagonistes de l'Angiotensine II.

L'Angiotensine II est connue pour être une hormone circulante pouvant agir également comme un neuropeptide au niveau du système nerveux central ainsi que l'indiquent notamment les demandes de brevets européennes EP 0465368 et EP 0503162, ou encore internationale WO.91/14367.

Il a récemment été mis en évidence qu'il existe en fait deux sous-types de récepteurs à l'Angiotensine II :
le récepteur AT₁ et le récepteur AT₂.

La recherche s'était d'abord intéressée à la mise en évidence d'antagonistes au récepteur AT₁ comme substances à activité anti-hypertensives.

On vient de montrer que les produits de formule (I) de la présente invention ont une affinité non seulement pour le récepteur AT₁ mais également pour le récepteur AT₂.

Les produits de formule (I) de la présente invention peuvent ainsi tout particulièrement faire l'objet d'une nouvelle utilisation pour le traitement d'affections résultant d'une stimulation anormale des récepteurs AT₁ et AT₂ de l'Angiotensine II.

La présente invention a ainsi pour objet la nouvelle utilisation pour la préparation de compositions pharmaceutiques destinées au traitement d'affections résultant d'une stimulation anormale des récepteurs AT₁ et AT₂ de l'angiotensine II, de produits de formule (I) 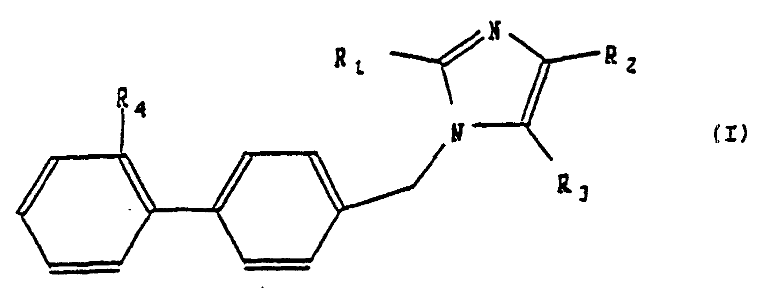 répondant aux formules suivantes :
- Acide 2-butyl α-hydroxy α-méthyl 4-(méthylthio) 1-[(2'-((((phénylméthoxy) carbonyl) amino) sulfonyl) (1,1'-biphényl) 4-yl) méthyl] 1H-imidazole 5-acétique,
- Acide 2-butyl α-hydroxy α-méthyl 4-(méthylthio) 1-[(2'-((((propylamino) carbonyl) amino) sulfonyl) (1,1'-biphényl) 4-yl) méthyl] 1H-imidazole 5-acétique,
- Acide 2-butyl α-hydroxy α-méthyl 4-(méthylthio) 1-[(2'-((((phénylméthyl) amino) carbonyl) amino) sulfonyl) (1,1'-biphényl) 4-yl) méthyl] 1H-imidazole 5-acétique,
- Acide 2-butyl 1-[(2'-(((((cyclohexylméthyl) amino) carbonyl) amino) sulfonyl) (1,1'-biphényl) 4-yl) méthyl] α-hydroxy α-méthyl 4-(méthylthio) 1H-imidazole 5-acétique,
- Acide 1-[(2'-(((((phénylméthyl) amino) sulfonyl) (1,1'-biphényl) 4-yl) méthyl] α-hydroxy 4-(méthylthio) 2-propyl 1H-imidazole 5-acétique,
- 1-[(2'-(((((cyclohexylméthyl) amino) carbonyl) amino) sulfonyl) (1,1'-biphényl) 4-yl) méthyl] 4-(méthylthio) 2-propyl 1H-imidazole 5-carboxylate de sodium,
- Acide 4-(méthylthio) 1-[(2'-(((((phénylméthyl) amino) carbonyl) amino) sulfonyl) (1,1'-biphényl) 4-yl) méthyl] 2-propyl 1H-imidazole 5-carboxylique,
- α-butyl α-hydroxy 4-(méthylthio) 1-[(2'-(((((phénylméthyl) amino) carbonyl) amino) sulfonyl) (1,1'-biphényl) 4-yl) méthyl] 2-propyl 1H-imidazole 5-acétate de sodium,
- Acide α-hydroxy 4-(méthylthio) α-phényl 1-((2'-(((((phénylméthyl) amino) carbonyl) amino) sulfonyl) (1,1'-biphényl) 4-yl) méthyl) 2-propyl 1H-imidazole 5-acétique,
lesdits produits de formule (I) étant sous toutes les formes isomères racémiques, énantiomères et diastéréoisomères possibles ainsi que les sels d'addition avec les acides minéraux et organiques ou avec les bases minérales et organiques desdits produits de formule (I) pour la préparation de compositions pharmaceutiques destinées au traitement d'affections résultant d'une stimulation anormale aux récepteurs AT₁ et AT₂ de l'Angiotensine II.

Les produits de formule (I) tels que définis ci-dessus sont décrits de façon générale dans les demandes de brevets européennes EP 465368 et EP 503162 et peuvent dont être préparés notamment ainsi qu'il est indiqué dans ces demandes de brevets européennes.

Le radical carboxy des produits de formule (I) peut être salifié par les groupements divers connus de l'homme du métier parmi lesquels on peut citer, par exemple : des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoique, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

L'invention a aussi particulièrement pour objet les produits de formule (I) répondant aux formules suivantes :
- Acide 2-butyl α-hydroxy α-méthyl 4-(méthylthio) 1-[(2'-((((phénylméthoxy) carbonyl) amino) sulfonyl) (1,1'-biphényl) 4-yl) méthyl] 1H-imidazole 5-acétique,
- Acide 2-butyl α-hydroxy α-méthyl 4-(méthylthio) 1-[(2'-((((propylamino) carbonyl) amino) sulfonyl) (1,1'-biphényl) 4-yl) méthyl] 1H-imidazole 5-acétique,
- Acide 2-butyl α-hydroxy α-méthyl 4-(méthylthio) 1-[(2'-((((phénylméthyl) amino) carbonyl) amino) sulfonyl) (1,1'-biphényl) 4-yl) méthyl] 1H-imidazole 5-acétique,
- Acide 2-butyl 1-[(2'-(((((cyclohexylméthyl) amino) carbonyl) amino) sulfonyl) (1,1'-biphényl) 4-yl) méthyl] α-hydroxy α-méthyl 4-(méthylthio) 1H-imidazole 5-acétique,
- Acide 1-[(2'-(((((phénylméthyl) amino) sulfonyl) (1,1'-biphényl) 4-yl) méthyl] α-hydroxy 4-(méthylthio) 2-propyl 1H-imidazole 5-acétique,
- 1-[(2'-(((((cyclohexylméthyl) amino) carbonyl) amino) sulfonyl) (1,1'-biphényl) 4-yl) méthyl] 4-(méthylthio) 2-propyl 1H-imidazole 5-carboxylate de sodium,
- Acide 4-(méthylthio) 1-[(2'-(((((phénylméthyl) amino) carbonyl) amino) sulfonyl) (1,1'-biphényl) 4-yl) méthyl] 2-propyl 1H-imidazole 5-carboxylique,
- α-butyl α-hydroxy 4-(méthylthio) 1-[(2'-(((((phénylméthyl) amino) carbonyl) amino) sulfonyl) (1,1'-biphényl) 4-yl) méthyl] 2-propyl 1H-imidazole 5-acétate de sodium,
- Acide α-hydroxy 4-(méthylthio) α-phényl 1-((2'-(((((phénylméthyl) amino) carbonyl) amino) sulfonyl) (1,1'-biphényl) 4-yl) méthyl) 2-propyl 1H-imidazole 5-acétique.

L'invention a également pour objet un procédé de préparation des produits de formule (I), telle que définie ci-dessus, caractérisé en ce que :
soit l'on soumet un composé de formule (II): 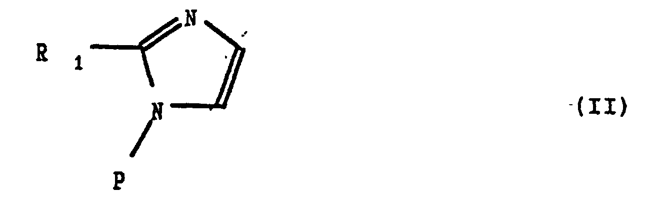 dans laquelle R ₁ a la signification indiquée ci-dessus et P représente un groupement protecteur de l'atome d'azote, à une réaction d'halogénation pour obtenir un composé de formule (III) : 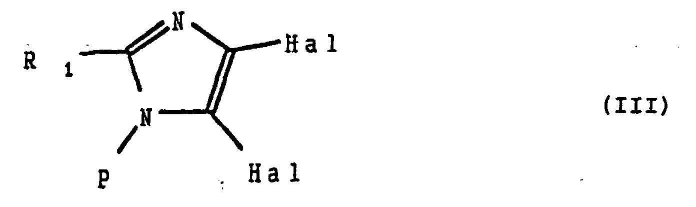 dans laquelle R ₁ et P ont les significations indiquées ci-dessus et Hal représente un atome d'halogène, que l'on soumet à une réaction d'échange halogène-métal sur l'un des atomes d'halogène puis à une réaction avec un composé de formule (IV_{c}) ou (IV_{d}) : 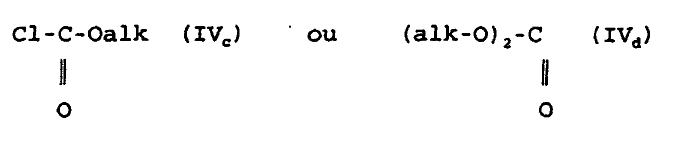 dans lesquelles alk représente un radical alkyle renfermant au plus 4 atomes de carbone,
   pour obtenir le composé de formule (V) : 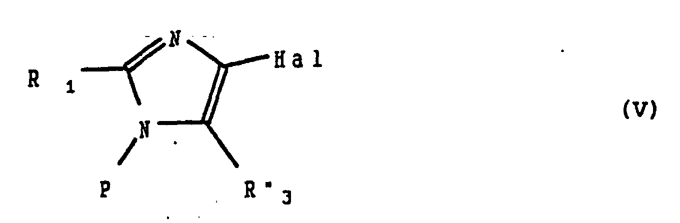 dans laquelle R ₁, P et Hal ont les significations indiquées ci-dessus et R"₃ représente CO-O-alk tel que défini ci-dessus, composé de formule (V) que l'on soumet à une réaction d'échange halogène-métal sur l'atome d'halogène puis à une réaction avec un composé de formule (IV ₐ) ou (IV _{b}) :

   (-S-R')₂ (IV ₐ) ou MeSO₂SR' (IV _{b})

   dans lesquelles SR' a la signification indiquée ci-dessus pour R₂, pour obtenir le composé de formule (VII) : 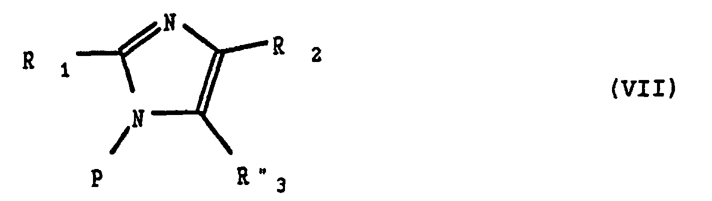 dans laquelle R ₁, R₂, R"₃ et P ont les significations indiquées ci-dessus,
   produit de formule (VII) dont on libère la fonction amine bloquée par P tel que défini ci-dessus, puis fait réagir avec un composé de formule (VIII) : 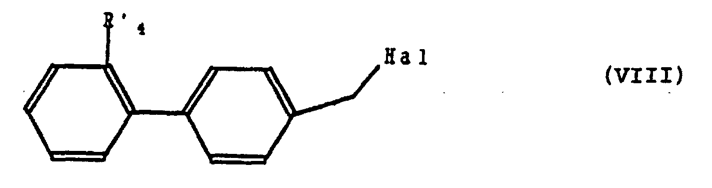 dans laquelle R'₄ a la signification indiquée ci-dessus pour R₄, dans laquelle les éventuelles fonctions réactives sont éventuellement protégées par des groupements protecteurs et Hal représente un atome d'halogène pour obtenir un produit de formule (I₁) : 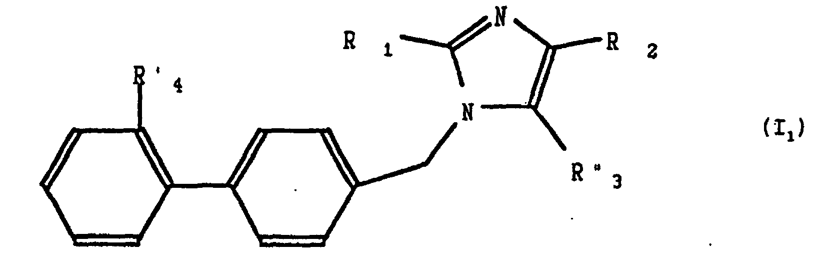 dans laquelle R ₁, R ₂, R"₃ et R'₄ ont les significations indiquées ci-dessus,
soit l'on soumet un composé de formule (IX) : 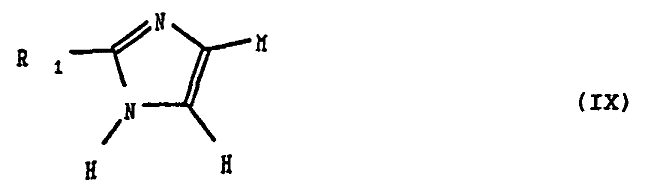 dans laquelle R ₁ a la signification indiquée ci-dessus et M représente un atome d'hydrogène ou le radical R ₂ qui a la signification indiquée ci-dessus,
   à une réaction avec le composé de formule (VIII) telle que définie ci-dessus, pour obtenir un produit de formule (X) : 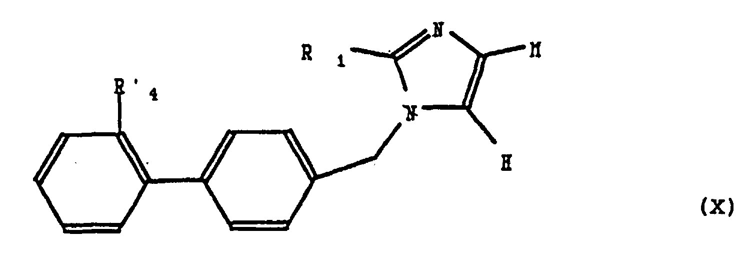 dans laquelle R ₁, M et R'₄ ont les significations indiquées ci-dessus,
   produits de formule (X) que lorsque M représente R ₂ tel que défini ci-dessus, l'on soumet à une réaction d'halogénation pour obtenir le produit de formule (XI) : 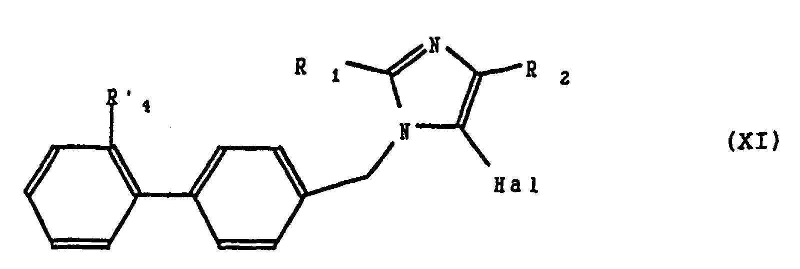 dans laquelle R ₁, R ₂, R'₄ et Hal ont les significations indiquées ci-dessus, que l'on soumet à une réaction d'échange halogène-métal puis à une réaction avec un composé de formule (XII) : 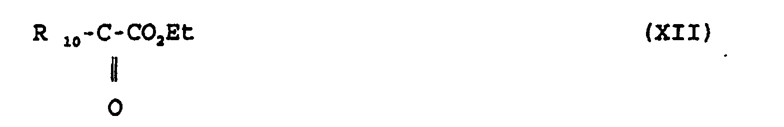 dans laquelle R ₁₀ a la signification telle que 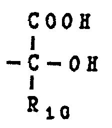 est un groupe R₃, pour obtenir un produit de formule (I₂) : 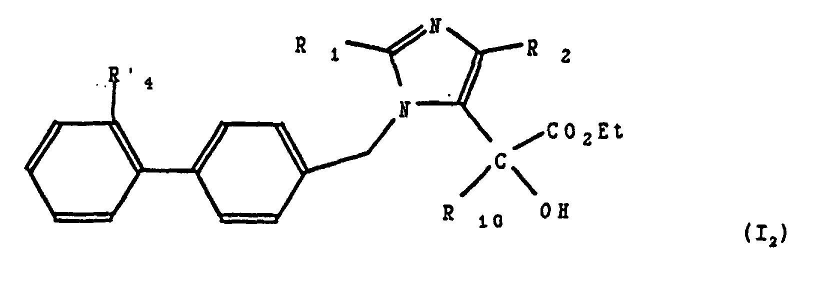 dans laquelle R ₁, R ₂, R'₄ et R ₁₀ ont les significations indiquées ci-dessus,
   que l'on soumet à une réaction de saponification pour obtenir le produit de formule (I₄) : 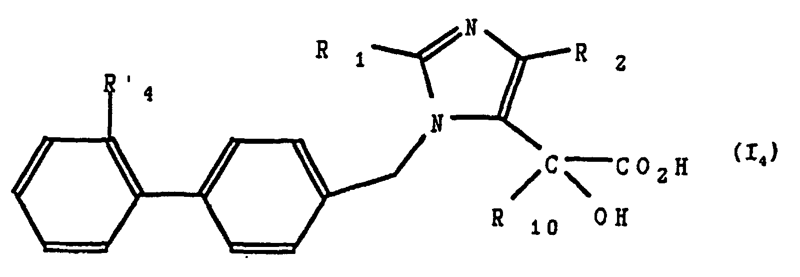 dans laquelle R ₁, R ₂, R'₄ et R ₁₀ ont les significations indiquées ci-dessus,
   produit de formule (X) que lorsque M représente un atome d'hydrogène, l'on soumet à une réaction d'halogénation pour obbtenir le produit de formule (XIV) : 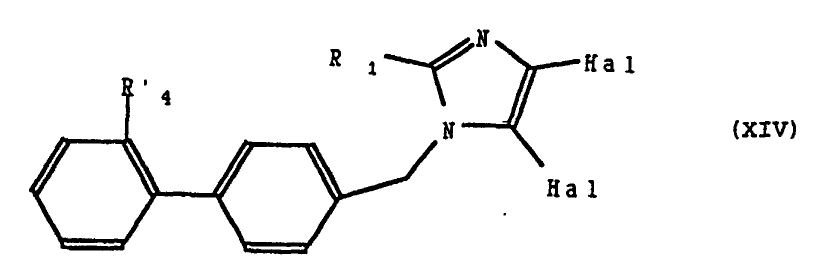 dans laquelle R ₁, R'₄ et Hal ont les significations indiquées ci-dessus, que l'on soumet à une réaction d'échange halogène métal puis à l'action d'un composé de formule (IV_{c}) ou (IV_{d}) telle que définie ci-dessus pour obtenir le produit de formule (XXI) : 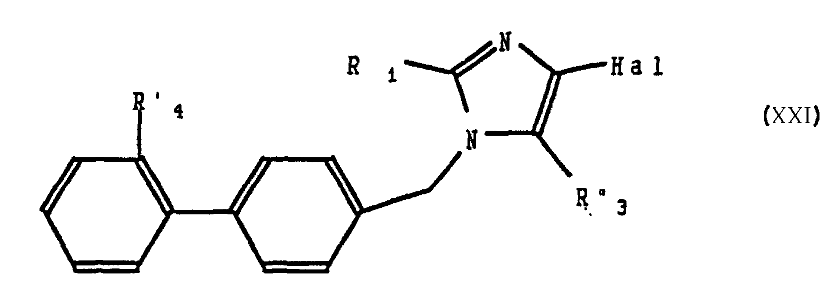 dans laquelle R ₁, R'₄, Hal et R"₃ ont les significations indiquées ci-dessus, produit de formule (XXI) que l'on soumet à une réaction d'échange halogène métal puis à l'action d'un composé de formule (IV ₐ) ou (IV _{b}), telle que définie ci-dessus, pour obtenir un produit de formule (I) : 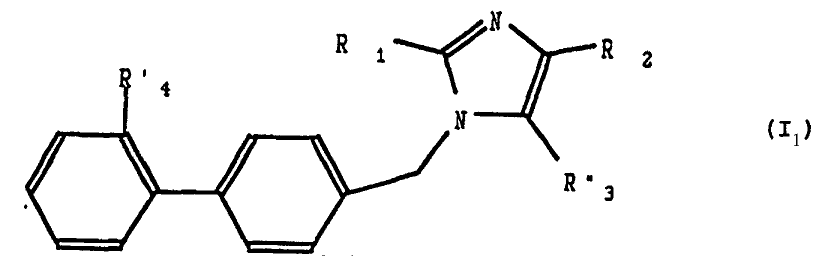 dans laquelle R ₁, R'₄, R ₂ et R"₃ ont les significations indiquées ci-dessus,
soit l'on soumet un composé de formule (XX) : 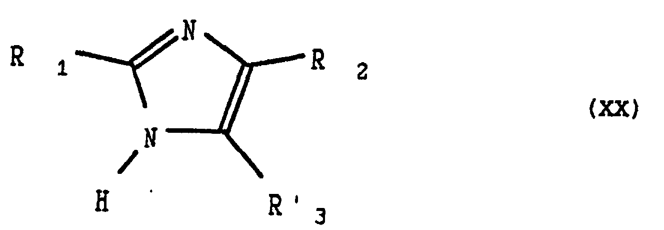 dans laquelle R ₁ et R ₂ ont les significations indiquées ci-dessus et R'₃ a la signification indiquée ci-dessus pour R₃ dans laquelle les éventuelles fonctions réactives sont éventuellement protégées par des groupements protecteurs, à une réaction avec le composé de formule (VIII) telle que définie ci-dessus, pour obtenir un produit de formule (I') : 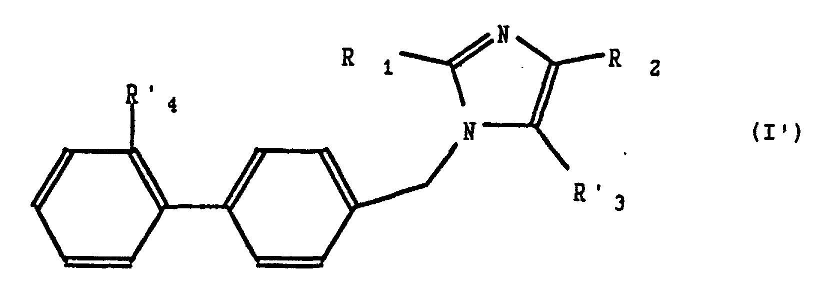 dans laquelle R ₁, R ₂, R'₃ et R'₄ ont les significations indiquées ci-dessus, produits de formules (I₄) et (I') qui peuvent être des produits de formule (I) et que, pour obtenir des ou d'autres produits de formule (I), l'on peut soumettre, si désiré et si nécessaire, ainsi que les produits de formule (I₁), à l'une ou plusieurs des réactions de transformations suivantes, dans un ordre quelconque :
   b) saponification de fonction ester en fonction acide,
   d) transformation de fonction cyano en fonction acide,
   e) transformation de fonction acide en fonction amide,
   g) transformation de fonction alcoxy en fonction hydroxyle,
   h) oxydation de fonction alcool en fonction acide,
   i) transformation d'un radical formyle en radical carbamoyle,
   r) transformation d'un carbamate en urée et notamment d'un sulfonylcarbamate en sulfonylurée,
   s) élimination des groupements protecteurs que peuvent porter les fonctions réactives protégées,
   t) salification par un acide minéral ou organique ou par une base pour obtenir le sel correspondant,
   u) dédoublement des formes racémiques en produits dédoublés, lesdits produits de formule (I) ainsi obtenus étant sous toutes les formes isomères possibles racémiques, énantiomères et diastéréoisomères.

Dans des conditions préférentielles de mise en oeuvre de l'invention, la réaction d'halogénation des composés de formule (II) et (X) telles que définies ci-dessus respectivement en composés de formule (III) et (XI) ou (XIV) telles que définies ci-dessus, peut être réalisée dans les conditions usuelles de l'homme du métier et notamment par bromation à l'aide de N-bromosuccinimide dans le dichlorométhane ou encore de brome dans l'acide acétique.

L'obtention du composé de formule (V) correspondant peut être réalisée par réaction du composé de formule (III) telle que définie ci-dessus avec un composé organo métallique tel que le n-butyllithium dans un solvant tel que le tétrahydrofuranne à une température,d'environ -78°C suivie de l'action d'un composé de formule (IV_{c}) ou (IV_{d}).

La réaction du produit de formule (V) telle que définie ci-dessus avec le composé de formule (IV ₐ) ou (IV _{b}), telle que définie ci-dessus, pour obtenir un composé de formule (VII) telle que définie ci-dessus peut être réalisée d'une manière identique en utilisant le n-butyllithium comme agent de métallation.

La fonction amine du composé de formule (VII) telle que définie ci-dessus, protégée par P tel que défini ci-dessus, peut être libérée dans les conditions usuelles connues de l'homme du métier et notamment lorsque P représente le radical -CH₂-O-(CH₂)₂-Si(CH₃)₃, par action de l'acide trifluoroacétique ou encore en présence d'ion fluorure.

Dans le produit de formule (VIII), Hal représente de préférence un atome de brome mais peut également représenter un atome de chlore ou d'iode.

La réaction du produit de formule (VIII) sur le produit de formule (VII) ou (IX) ou (XX), peut être réalisée dans un solvant tel que par exemple le diméthylformamide ou encore le diméthylacétamide, le tétrahydrofuranne, le diméthoxyéthane ou le diméthylsulfoxyde au reflux du solvant ou à la température ambiante, de préférence sous agitation ; la réaction est réalisée de préférence en présence d'une base telle que par exemple l'hydrure de sodium ou de potassium ou encore du carbonate de sodium ou de potassium, du méthylate ou éthylate ou tert-butylate de sodium ou de potassium.

L'obtention du composé de formule (I₂) telle que définie ci-dessus est réalisée par action, sur le dérivé magnésien du composé de formule (XI), du composé de formule (XII) telle que définie ci-dessus dans un solvant tel que par exemple le tétrahydrofuranne ou le toluène.

Le dérivé magnésien du composé de formule (XI) est réalisée par action du composé de formule (XI) telle que définie ci-dessus dans laquelle Hal peut par exemple représenter un atome de brome, avec un composé magnésien comme par exemple le chlorure d'isopropyle magnésium, dans un solvant tel que par exemple le toluène.

La réaction de saponification du produit de formule (I₂) telle que définie ci-dessus, en produit de formule (I₄) telle que définie ci-dessus, peut être réalisée selon les méthodes usuelles connues de l'homme du métier, telles que par exemple en présence de soude ou de potasse ou encore de carbonate de cesium, dans un solvant tel que le méthanol ou l'éthanol le dioxane ou le diméthoxyéthane.

La réaction de transformation du produit de formule (XIV) telle que définie ci-dessus, en produit de formule (XXI) telle que définie ci-dessus puis en produit de formule (I₁), telle que définie ci-dessus, peut être réalisée dans les mêmes conditions que celles définies pour l'obtention des produits de formules (V) et (VII) telles que définies ci-dessus, à partir du produit de formule (III) telle que définie ci-dessus.

Selon les valeurs de R'₃ et R'₄, les produits de formules (I₄) et (I') constituent ou non des produits de formule (I) et peuvent donner des produits de formule (I), ou être transformés en d'autres produits de formule (I) en étant soumis à une ou plusieurs des réactions b) à u) indiquées ci-dessus.

Ainsi les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals ou de thiocétals cycliques ou non cycliques tels que le diméthyl ou diéthylcétal ou l'éthylène dioxycétal, ou le diéthylthiocétal ou l'éthylènedithiocétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante :
- les fonctions acide peuvent être protégées par exemple sous forme d'esters formés avec les esters facilement clivables tels que les esters benzyliques ou ter butyliques ou des esters connus dans la chimie des peptides.

Les réactions auxquelles les produits de formules (I₁), (I₄) et (I'), telles que définies ci-dessus peuvent être soumis, si désiré ou si nécessaire, peuvent être réalisées, par exemple, comme indiqué ci-après.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
d) Les éventuelles fonctions cyano des produits décrits ci-dessus peuvent être, si désiré, transformées en fonction acide dans les conditions usuelles connues de l'homme du métier par exemple par une double hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.
e) La réaction de transformation de fonction acide en fonction amide peut notamment être réalisée par formation d'abord d'un chlorure d'acide selon les conditions usuelles connues de l'homme du métier et par exemple par action de SOCl₂ puis amidification comme indiqué ci-dessus, ou encore par amidification directe de l'acide ci-dessus.
g) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
h) Les éventuelles fonctions alcool des produits décrits ci-dessus peuvent être, si désiré, transformées en fonction acide par oxydation dans les conditions usuelles connues de l'homme du métier telles que par exemple par action du réactif de Jones pour accéder aux acides.
i) Les réactions de transformation de radical formyle en radical carbamoyle sont réalisées notamment pour R₄ selon les conditions usuelles connues de l'homme du métier, telles que par exemple passage par le céto nitrile et déplacement par une amine (Chem. Comm. 1971, p. 733).
r) la réaction de transformation d'un carbamate en urée et notamment d'un sulfonylcarbamate en sulfonylurée, peut être réalisée par exemple au reflux d'un solvant comme par exemple le toluène en présence de l'amine adéquate.
s) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
   On trouvera une liste de différents groupements protecteurs utilisables par exemple dans le brevet BF 2 499 995.
t) Les produits décrits ci-dessus peuvent, si désiré, faire l'objet de réactions de salification par exemple par un acide minéral ou organique ou par une base minérale ou organique selon les méthodes usuelles connues de l'homme du métier.
u) Les éventuelles formes optiquement actives des produits décrits ci-dessus peuvent être préparées par dédoublement des racémiques selon les méthodes usuelles connues de l'homme du métier.

Des illustrations de telles réactions définies ci-dessus sont données dans la préparation des exemples décrits ci-après .

Certains produits de départ de formules (II), (IX) et (XX) sont connus et peuvent être préparés par exemple comme indiqué dans le brevet européen EP 168 950.

D'autres produits de départ de formules (II), (IX) et (XX) peuvent en particulier être préparés comme indiqué dans le brevet européen EP 0465368 ou dans les préparations 1 à 5 décrites ci-après.

Certains produits de départ de formules (II), (IX) et (XX) sont commerciaux tels que par exemple :
les produits de formule (II) suivants :
   - le 2-phénylimidazole
   - le 2-méthoxyméthylimidazole
   - le 2-propylimidazole
   - le 2-isopropylimidazole
   - le 2-éthylimidazole
   - le 2-méthylimidazole
les produits de formule (IX) suivants :
   - le 4-méthyl 2-phénylimidazole
   - le 2,4-diméthylimidazole
   - le 2-éthyl 4-méthylimidazole.

Des exemples de produits commerciaux de formule (XX) sont donnés dans les brevets EP 0465368 ou EP 0503162.

On peut encore notamment préparer certains produits de formules (II), (IX) et (XX) à partir d'autres produits de formule (II) ou (IX) par exemple en les soumettant à une ou plusieurs des réactions décrites aux points b) à u) ci-dessus réalisées comme indiquées ci-dessus.

Certains produits de formules (IX) et (XX) peuvent également être obtenus par monohalogénation du produit de formule (II) tel que défini ci-dessus en produit de formule (P₁) : 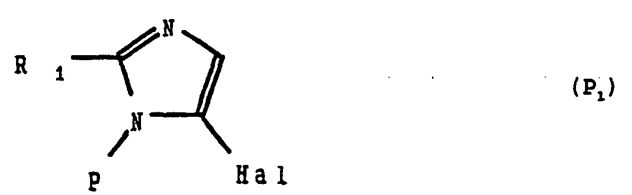 dans lequel R ₁ et P ont les significations indiquées ci-dessus pour le produit de formule (II), produit de formule (P₁) que l'on peut faire réagir après échange suivant la réaction halogène métal connue de l'homme du métier avec l'électrophile adapté, suivant les méthodes connues de l'homme du métier et notamment ainsi qu'il a été décrit ci-dessus pour passer du produit de formule (III) au produit de formule (V). Par le même processus, certains produits de formules (IX) et (XX) peuvent également être obtenus à partir du produit de formule (III) telle que définie ci-dessus. On peut encore noter que le produit de formule : 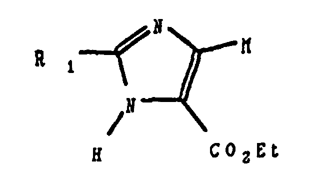 dans laquelle R ₁ et M ont les significations indiquées ci-dessus, décrit dans EP 0465368, peut être soumis à une réaction de saponification thermique puis à une décarboxylation pour obtenir un produit de formule (IX) telle que définie ci-dessus.

Une telle illustration est donnée dans la partie expérimentale décrite ci-après.

Les composés de départ de formule (VIII) peuvent être disponibles dans le commerce ou peuvent être préparés selon les méthodes usuelles connues de l'homme du métier.

Les produits de départ de formules (IVₐ), (IV_{b}), (IV_{c}), (IV_{d}), (XII) et (XV) sont commerciaux notamment les produits de formule (IVₐ) comme le méthyl disulfide,
le produit de formule (IV_{b}) comme le méthyl méthanethiosulfonate,
les produits de formule (IV_{c}) comme le méthyl chloroformate, le benzyl chloroformate, l'isobutyl chloroformate, l'éthyl chloroformate, le N-propyl chloroformate,
les produits de formule (IV_{d}) comme le diméthyl carbonate, le diéthyl carbonate,
les produits de formule (XII) comme l'éthyl thiophène 2-glyoxylate, l'éthyl 3-méthyl 2-oxobutyrate, l'éthyl phényl glyoxylate, le méthyl pyruvate, le méthyl benzoylformate, les produits de formule (XV) comme le méthyl isocyanate, le 2-carbométhoxyphényl isocyanate, le benzyl isocyanate, le cyclohexyl isocyanate, le N-propyl isocyanate, l'allyl isocyanate, le phényl isocyanate.

Un procédé de préparation de certains produits de formule (VIII) est notamment décrit dans le brevet européen EP 0465368.

Des exemples de préparation de composés de formule (VIII) sont également décrits dans la littérature et des exemples en sont donnés notamment dans le brevet US 4,880,804 ou par exemple dans la référence Chemistry and Industry 7 september 1987 HOWARD and COLQUHOUN pp. 612-617.

Les composés de formule (I) tels que définis ci-dessus ainsi que leurs sels d'addition avec les acides présentent d'intéressantes propriétés pharmacologiques.

Les produits sont doués de propriétés antagonistes pour le récepteur à l'angiotensine II et sont ainsi notamment inhibiteurs des effets de l'angiotensine II, en particulier de l'effet vasoconstricteur et également de l'effet trophique au niveau des myocytes.

Ces propriétés justifient leur application en thérapeutique et l'invention a également pour objet à titre de médicaments, les produits de formule (I) telle que définie ci-dessus, lesdits produits de formule (I) étant sous toutes les formes isoméres possibles racémiques ou optiquement actives, ainsi que les sels d'addition avec les acides minéraux ou organiques pharmaceutiquement acceptables desdits produits de formule (I).

Les médicaments, objet de l'invention, peuvent être utilisés dans le traitement des affections cardiovasculaires associées à une altération de la vasomotricité ou de la volémie : infarctus du myocarde et ses conséquences, insuffisance cardiaque, insuffisance rénale, angine de poitrine, hyperaldostéronisme, hypertension artérielle et ses conséquences. Ces médicaments, objet de l'invention, pourraient également être utilisés pour le traitement du glaucome, de l'athérosclérose et de différents types de spasmes viscéraux, ainsi qu'à titre de substances protectrices neuronales ou encore dans la prévention des resténoses post-angioplastie.

Notamment les médicaments, objet de l'invention peuvent être utilisés pour leurs effets anti-hypertrophique et antifibrotique aux niveaux cardiaque et vasculaire. Tout particulièrement, ils peuvent être utilisés pour le traitement et la prévention des désordres cardiovasculaires associés au diabète.

Ils peuvent également être utilisés dans le traitement de certains désordres gastro-intestinaux, gynécologiques et en particulier pour un effet relaxant au niveau de l'utérus.

Les médicaments, objet de l'invention peuvent également être utilisés dans le traitement des troubles de la mémoire et des fonctions cognitives, ainsi que de l'anxiété.

L'invention s'étend aux compositions pharmaceutiques contenant à titre de principe actif l'un au moins des médicaments tels que définis ci-dessus.

Les produits de formule (I) de la présente invention ont donc une affinité non seulement pour le récepteur AT₁ mais également pour le récepteur AT₂ de l'Angiotensine II.

L'invention a donc particulièrement pour objet l'utilisation des produits de formule (I) telle que définie ci-dessus, pour la préparation de compositions pharmaceutiques destinées au traitement d'affections résultant d'une stimulation anormale des récepteurs AT₁ et AT₂ de l'Angiotensine II.

L'avantage que présentent de tels produits de formule (I), qui possèdent une affinité à la fois pour le récepteur AT₁ et pour le récepteur AT₂ est par le blocage simultané de ces deux récepteurs, d'augmenter l'efficacité c'est-à-dire les effets protecteurs, résultant de l'administration de tels produits vis-àvis des organes touchés notamment le coeur, les vaisseaux et les reins et également d'élargir les indications notamment pour les organes non cardiovasculaires tels que l'appareil urogénital.

Les taux circulants de l'Angiobensine II étant augmentés par un phénomène de rétro-contrôle lors du blocage du récepteur AT₁, le blocage simultané du récepteur AT₂ permettrait une meilleure efficacité à long terme dans le traitement notamment de l'hypertension artérielle et la prévention des complications en particulier les hypertrophies cardiaques et vasculaires ainsi que le développement de fibroses au niveau des organes cibles.

On peut également souligner l'amélioration des propriétés cognitives des produits montrant une affinité à la fois pour les récepteurs AT₁ et AT₂.

L'invention a ainsi plus particulièrement pour objet l'utilisation des produits de formule (I), telles que définies ci-dessus, pour la préparation de compositions pharmaceutiques destinées au traitement de l'hypertension artérielle, de l'insuffisance cardiaque et des resténoses post-angioplastie.

L'invention a tout particulièrement pour objet l'utilisation des produits de formule (I), telles que définies ci-dessus, pour la préparation de compositions pharmaceutiques destinées au traitement de l'insuffisance rénale.

L'invention a également pour objet l'utilisation des produits de formule (I), telles que définies ci-dessus, pour la préparation de compositions pharmaceutiques destinées au traitement et à la prévention des désordres cardiovasculaires associés au diabète.

Les compositions pharmaceutiques indiquées ci-dessus peuvent être administrées par voie buccale, rectale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemplè, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 100 mg par jour chez l'adulte, par voie orale.

Les exemples suivants illustrent l'invention sans toutefois la limiter.

### PREPARATION 1 : 2-butyl α-hydroxy α-méthyl 1-[(2'-((((diméthylamino) méthylène) amino) sulfonyl) (1,1'-biphényl) 4-yl) méthyl] 4-(méthylthio) 1H-imidazole 5-acétate d'éthyle

### Stade A : 4'-[(2-butyl 4-(méthylthio) 1H-imidazol 1-yl) méthyl] N-[(diméthylamino) méthylène] (1,1'-biphényl) 2-sulfonamide

### a) 2-butyl 4-(méthylthio) 1H-imidazole

On introduit 760 mg de 2-n-butyl 4-méthylthio imidazole 5-carboxylate d'éthyle, préparé ainsi qu'il est décrit dans la demande de brevet européen EP 0465368, dans 15 cm³ de NaOH(2N). On porte à reflux et agite durant 24 heures. Après refroidissement, on dilue par 50 cm³ H₂O, extrait par 3 x 20 cm³ CH₂Cl₂, lave par 20 cm³ H₂O et sèche. On obtient 535 mg de produit attendu.
F = 64°C.

| Spectre IR (CHCl₃) | |
|---|---|
| Absence de C=0 | |
| =C-NH | 3452 cm⁻¹ |
| Système conjugué | 1596 - 1502 cm⁻¹ |

### b) 4'-[(2-butyl 4-(méthylthio) 1H-imidazol 1-yl) méthyl] N-[(diméthylamino) méthylène] (1,1'-biphényl) 2-sulfonamide

On dissout 5 g de 2-butyl 4-(méthylthio) 1H-imidazole dans 120 cm³ de THF. Puis on ajoute lentement à la solution orangée obtenue 1,55 g d'hydrure de sodium à 50 % en dispersion dans l'huile. La température s'élève jusqu'à 25° C. On agite 30 mn à cette température puis on introduit 14 g de 4'-bromométhyl N-[(diméthylamino) méthylène] (1,1'-biphényl) 2-sulfonamide. On agite à température ambiante jusqu'à fin d'évolution, soit pendant environ 3 heures. On reprend à l'eau, extrait à l'acétate d'éthyle, sépare par chromatographie sur silice en éluant à l'acétate d'éthyle puis empâte à l'éther iso, filtre et sèche. On obtient ainsi 9,35 g du produit attendu (cristaux incolores). PF = 148° C.

| Spectre IR : CHCl₃ | |
|---|---|
| Absence de =C-NH- | |
| -SO₂-N=C-N〈 | 1627 cm⁻¹ |
| aromatique, hétéroaromatique | 1593-1564-1516-1500 cm⁻¹ |

### Stade B : 4'-[(5-bromo 2-butyl 4-(méthylthio) 1H-imidazol 1-yl) méthyl] N-[(diméthylamino) méthylène] (1,1'-biphényl) 2-sulfonamide

On dissout 10,4 g du produit obtenu au stade A, ci-dessus, dans 450 cm³ de CH₂Cl₂ et ajoute 3,9 g de N-bromosuccinimide.

On agite environ 15 mn à température ambiante, lave à l'eau et à l'eau salée, décante, sèche, filtre et chasse le solvant sous vide à 50°C.

On empâte à l'éther iso, filtre et sèche. On obtient 11,8 g de produit attendu (cristaux incolores) F = 158°C.

| Spectre IR : CHCl₃ | |
|---|---|
| - N=CH-N〈 | 1628 cm-1 |
| aromatique et hétéroaromatique | 1592-1568 cm⁻¹ |

| Microanalyse : | |
|---|---|
| | Br |
| % calculé | 14,54 |
| % trouvé | 14,4-14,7 |

### Stade C : 2-butyl α-hydroxy α-méthyl 1-[(2'-((((diméthylamino) méthylène) amino) sulfonyl) (1,1'-biphényl) 4-yl) méthyl] 4-(méthylthio) 1H-imidazole 5-acétate d'éthyle

On dissout 11,8 g du produit obtenu au stade B ci-dessus dans 160 cm³ de THF. Puis on ajoute sans dépasser 25°C, 17,5 cm³ d'une solution 1M de chlorure d'isopropyl magnésium en solution dans l'éther. Après environ 30 mn d'agitation à température ambiante, on ajoute lentement 4 cm³ de pyruvate d'éthyle. On agite environ 1 heure à température ambiante, ajoute 1 cm³ de pyruvate d'éthyle et poursuit l'agitation pendant encore environ 1 heure.

On reprend par 200 cm³ de NH₄Cl en solution à 10 % et extrait à l'acétate d'éthyle. On sèche, concentre jusqu'à ∼ 200 cm³, filtre et sèche les cristaux obtenus.

On obtient 6 g de produit attendu (cristaux incolores). F = 208-210°C.

| Spectre IR : CHCl₃ | |
|---|---|
| OH complexe | ∼ 3530 cm⁻¹ |
| C=O | 1722 cm⁻¹ |
| C=N | 1626 cm⁻¹ |
| Hétérocycle + aromatique | 1565, 1518 cm⁻¹ |

| Spectre UV : | |
|---|---|
| 1) Dans EtOH | |
| infl. 274 nm | ε = 3100 |
| infl. 231 nm | ε = 23000 |

| 2) Dans EtOH - HCl N/10 | |
|---|---|
| infl. 228 nm | ε = 30000 |
| infl. 273 nm | ε = 3400 |

### PREPARATION 3 : 1-[(2'-(aminosulfonyl) (1,1'-biphényl) 4-yl) méthyl] 4-(méthylthio) α-oxo 2-propyl 1H-imidazole 5-acétate d'éthyle

### Stade A : 4,5-dibromo 2-n-propyl 1-[(2-(triméthylsilyl) éthoxy) méthyl] 1H-imidazole

On procède comme au stade A.de la préparation 2, en remplaçant 2-méthyl 1H-imidazole par le 2-propyl 1H-imidazole.

Le produit est obtenu par 2 réactions consécutives.

### 1) Etape de protection de la 2-propyl imidazole

On dissout 30 g de 2-propyl imidazole dans 500 cm³ de THF et on ajoute par petites fractions 12,5 g d'hydrure de sodium à 50 % dans l'huile.

On ajoute alors dans le milieu réactionnel 53 cm³ de chlorure de SEM, on hydrolyse par du THF à 20 % H₂O.

On obtient ainsi 57,3 g de produit protégé.

### 2) Bromation

Le produit protégé obtenu ci-dessus est dissous dans 500 cm³ de CH₂Cl₂. On ajoute 93,5 g de N-bromo succinimide et obtient 93,7 g de produit attendu.

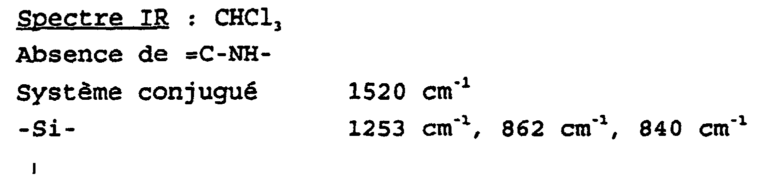

### Stade B : 2-n-propyl 1-[(2-(triméthylsilyl) éthoxy) méthyl] 5-(méthylthio) α-oxo 1H-imidazole 4-acétate d'éthyle

On introduit, sous atmosphère anhydre, 36,7 g du produit obtenu au stade A ci-dessus, dans 200 cm³ de THF, on ajoute à - 78°C 63,7 cm³ de n-butyl lithium 1,5 Molaire dans l'hexane puis 8,62 cm³ de diméthyl disulfure, on laisse remonter à température ambiante. Puis on ajoute comme précédemment à - 78°C 63,7 cm³ de n-butyl lithium 1,5 Molaire dans l'hexane suivi de 55 cm³ d'oxalate d'éthyle. On obtient 11,75 g de produit attendu.

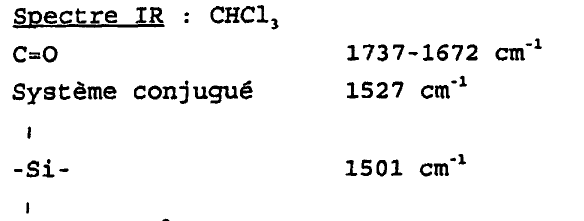

### Stade C : 2-n-propyl 5-(méthylthio) α-oxo 1H-imidazole 4-acétate d'éthyle

On procède comme au stade C de la préparation 2 à partir de 11 g du produit obtenu au stade B ci-dessus dans 200 cm³ de CH₂Cl₂, et 40 cm³ d'acide trifluoroacétique. On obtient 7,15 g de produit attendu.

| Spectre IR : CHCl₃ | |
|---|---|
| =C-NH | 3413 cm⁻¹ |
| C=O | 1714-1633 cm⁻¹ |
| Système conjugué | 1524-1492 cm⁻¹ |

### Stade D : 2-n-propyl 4-(méthylthio) 1-[(2'-((((diméthylamino) méthylène) amino) sulfonyl) (1,1'-biphényl) 4-yl) méthyl] α-oxo 1H-imidazole 5-acétate d'éthyle

On procède comme au stade D de la préparation 2 à partir de 7 g du produit obtenu au stade C ci-dessus dans 100 cm³ de DMF et 7,5 g de carbonate de potassium et 15,6 g de 4'-(bromométhyl) N-[(diméthylamino) méthylène] (1,1'-biphényl) 2-sulfonamide. On obtient ainsi 7,83 g de produit attendu.

| Spectre IR : CHCl₃ | |
|---|---|
| Absence de =C-NH | |
| C=O ester | 1735 cm⁻¹ |
| Autre C=O C=N | 1630 cm⁻¹ (F) |

### Stade E : 1-[(2'-(aminosulfonyl) (1,1'-biphényl) 4-yl) méthyl] 2-n-propyl 4-(méthylthio) α-oxo 1H-imidazole 5-acétate d'éthyle

On procède comme au stade A de l'exemple 2 à partir de 7,8 g du produit obtenu au stade D ci-dessus, dans 100 cm³ d'éthanol et 30 cm³ de HCl concentré et obtient ainsi 3,6 g de produit attendu.

| Spectre IR CHCl₃ | |
|---|---|
| - NH₂ | 3443-3343 cm⁻¹ |
| C=O | 1734-1627 cm⁻¹ |
| Aromatique | 1593 cm⁻¹ |
| Hétéroatome | 1565 cm⁻¹ |
| NH₂ def . | 1542 cm⁻¹ |

### PREPARATION 5 : 1-[(2'-(aminosulfonyl) (1,1'-biphényl) 4-yl) méthyl] 4-(méthylthio) 2-propyl 1H-imidazole 5-carboxylate d'éthyle

### Stade A : cyano-[(1-oxobutyl) amino] acétate d'éthyle

On mélange 5 g d'éthyl (hydroxyimino) cyanoacétate, 40 cm³ de tétrahydrofuranne, 11,5 cm³ d'anhydride butyrique et 2,5 g de platine et agite en atmosphère d'hydrogène jusqu'à saturation. On filtre, rince par 5 x 15 cm³ d'éther éthylique, évapore l'éther, ajoute peu à peu 200 cm³ d'essence G, essore, lave avec 3 x 10 cm³ d'essence G et sèche à environ 75°C. On concentre à ∼ 10 cm³, ajoute 50 cm³ d'essence G, laisse cristalliser 30 mn à température ambiante, essore, lave avec 3 x 3 cm³ d'essence G et sèche à environ 75°C. On obtient 5,73 g de produit. F = 110°C.

### Recristallisation pour analyses :

On dissout 540 mg du produit obtenu dans 50 cm³ d'éther isopropylique à reflux, filtre, concentre, laisse environ 1 h au repos à température ambiante, essore, lave à l'éther isopropylique et sèche. On obtient 440 mg de produit attendu. F = 110°C.

| Microanalyse pour C₉H₁₄N₂O₃ = 198,22 | | | | |
|---|---|---|---|---|
| | C | H | N | O |
| % calculé | 54,53 | 7,12 | 14,13 | 24,22 |
| % trouvé | 54,5 | 7,2 | 14,0 | |

| Spectre IR CHCl₃ | |
|---|---|
| =C-NH | ∼ 3430 cm⁻¹ |
| -C≡N | ∼ 2245 cm⁻¹ |
| C=O | 1758 cm⁻¹ ester |
| | 1692 cm⁻¹ amide |
| Amide II | 1492 cm⁻¹ |

### Stade B : 3-amino 2-[(1-oxobutyl) amino] 3-(méthylthio) 2-propenoate d'éthyle

A une solution de 20 g de nitrile obtenu au stade A ci-dessus, dans 400 ml d'éthanol, on ajoute 1,4 ml de triéthylamine, on refroidit à environ -10°C et introduit environ 22 g de méthylmercaptan par barbotage. On agite environ 72 heures à 0°C. On élimine l'excès de méthanethiol chasse l'éthanol, empâte à l'essence G, filtre et sèche. On obtient 24,3 g de produit attendu (cristaux incolores).
F_{K115} = 120-124°C

| Microanalyse pour C₁₀H₁₈N₂O₃S = 246,33 | | | | | |
|---|---|---|---|---|---|
| | C | H | N | S | O |
| % calculé | 48,76 | 7,37 | 11,37 | 13,02 | 19,49 |
| % trouvé | 48,6 | 7,5 | 11,4 | 12,6 | - |

| Spectre IR CHCl₃ | |
|---|---|
| =C-NH₂ | 3500, 3412 cm⁻¹ |
| =C-NH | 3365, 3275 cm⁻¹ |
| C=O complexe | 1665 cm⁻¹ |
| C=C et NH₂ déf. | 1592 cm⁻¹ |
| Amide II | 1488 cm⁻¹ |

| Spectre UV dans EtOH | |
|---|---|
| Max. 220 nm | ε = 5500 |
| Max. 291-292 | ε = 19400 |

### Stade C : 4-(méthylthio) 2-propyl 1H-imidazole 5-carboxylate d'éthyle

A 20,1 g de pentachlorure de phosphore dans 300 cm³ de chlorure de méthylène, refroidis à environ -70°C on ajoute une solution de 12,9 g de 4-diméthylaminopyridine dans 90 cm³ de chlorure de méthylène.

On maintient encore environ 15 mn à environ -70°C puis on introduit une solution de 12 g du produit obtenu au stade B ci-dessus dans 120 cm³ de chlorure de méthylène. On laisse revenir à température ambiante et maintient sous agitation pendant environ 22 heures.

On verse le mélange réactionnel dans 2,5 1 d'eau + glace et neutralise par addition d'environ 60 g de bicarbonate de sodium. On agite encore environ 30 mn, on décante et on extrait avec 500 cm³ de CH₂Cl₂. On lave à l'eau salée, sèche et chasse le solvant à environ 50°C. On purifie par chromatographie sur silice avec pour éluants CH₂Cl₂-ACOEt (90-10) puis CH₂Cl₂-ACOEt (80-20). On chasse les solvants à environ 50°C, empâte à l'essence G, filtre et sèche. On obtient 7,4 g de produit attendu (cristaux incolores). F_{K95} = 85°C.

| Microanalyse | | | | | |
|---|---|---|---|---|---|
| Concordance pour C₁₀H₁₆N₂O₂S = 228,32 | | | | | |
| | C | H | N | S | O |
| % calculé | 52,61 | 7, 06 | 12,27 | 14,04 | 14,02 |
| % trouvé | 52,7 | 7,3 | 12,2 | 14,0 | |

| Spectre IR CHCl₃ | |
|---|---|
| =C-NH | 3440 - 3260 cm⁻¹ |
| C=O Complexe max. | ∼ 1672 cm⁻¹ |
| Hétérocycle | 1542 - 1498 cm⁻¹ |

| Spectre UV dans EtOH | |
|---|---|
| Max. 213-214 nm | ε = 14500 |
| Infl. 229 nm | ε = 7200 |
| Max. 286 nm | ε = 12200 |

| Spectre UV dans EtOH/HCl N/10 | |
|---|---|
| Max. 238 nm | ε = 6800 |
| Max. 277 nm | ε = 9600 |
| par retour basique --> max. 296 nm. | |

### Stade D : 1-[(2'-((((diméthylamino) méthylène) amino) sulfonyl) (1,1'-biphényl) 4-yl) méthyl] 4-(méthylthio) 2-propyl 1H-imidazole 5-carboxylate d'éthyle

On dissout 8,1 g du produit obtenu au stade C ci-dessus, dans 80 cm³ de diméthylformamide et ajoute 16,1 g de 4'-(bromométhyl) N-[(diméthylamino) méthylène] (1,1'-biphényl) 2-sulfonamide et 4,9 g de carbonate de potassium. On agite à température ambiante pendant environ 24 heures. On chasse le DMF à 50°C, empâte à l'eau, filtre, lave à l'eau et sèche à environ 60°C.

On empâte dans 240 cm³ d'ACOEt, sous agitation pendant environ 30 mn à environ 50°C, ajoute 160 cm³ d'hexane, filtre et sèche. On obtient 14 g de produit attendu (cristaux incolores). F_{K163} : 182°C.

| Spectre IR CHCl₃ | |
|---|---|
| Absence de =C-NH | |
| C=O | 1690 cm⁻¹ |
| C=N | 1628 cm⁻¹ |
| Hétérocycle | 1504 - 1565 cm⁻¹ |

| Spectre UV dans EtOH | |
|---|---|
| Infl. 230 nm | ε = 32000 |
| Max. 287 nm | ε = 15500 |

### Stade E : 1-[(2'-(aminosulfonyl) (1,1'-biphényl) 4-yl) méthyl] 4-(méthylthio) 2-propyl 1H-imidazole 5-carboxylate d'éthyle

On mélange 11,2 g du produit obtenu au stade D, ci-dessus avec 200 cm³ d'éthanol et 100 cm³ d'acide chlorhydrique concentré. On chauffe au reflux pendant environ 2 heures. On évapore l'éthanol, dilue par addition de 400 cm³ d'eau, alcalinise sous agitation par addition de lessive de soude et extrait à l'acétate d'éthyle. On lave à l'eau et à l'eau salée, sèche, filtre et chasse le solvant à environ 50°C.

On purifie par chromatographie sur silice avec pour éluant ACOEt 60 Hexane 40. On obtient 9,3 g de produit attendu (cristaux incolores). F_{K135} = 130-132°C.

| Spectre IR CHCl₃ | |
|---|---|
| Absence de produit de départ | |
| C=O | 1689 cm⁻¹ |
| NH₂ | 3444 - 3340 cm⁻¹ |
| Système conjugué + aromatique NH₂ déf. | 1618 - 1590 - 1560 - 1540 - 1508 cm⁻¹ |

| Spectre UV dans EtOH | |
|---|---|
| Infl. 210 nm | ε = 45000 |
| Infl. 234 nm | ε = 17000 |
| Max. 286 nm | ε = 15000 |

### EXEMPLE 1 : 1-[(2'-(aminosulfonyl) (1,1'-biphényl) 4-yl) méthyl] 2-butyl α-hydroxy α-méthyl 4-(méthylthio) 1H-imidazole 5-acétate d'éthyle

On introduit 1,24 g du produit obtenu à la préparation 1, 30 cm³ d'éthanol à 95 et 10 cm³ d'HCl concentré.

On chauffe au reflux jusqu'à transformation totale, la solution obtenue soit pendant environ 2 heures.

On refroidit, dilue à l'eau, alcalinise par addition de NaOHc, sature la phase aqueuse par K₂CO₃, extrait à l'AcOEt, puis purifie par chromatographie sur silice avec comme solvant AcOEt. On obtient 930 mg de produit attendu (résine jaune paille).

| Spectre IR CHCl₃ | |
|---|---|
| Absence de -SO₂-N=CH-N〈 | |
| OH | ∼ 3520 cm⁻¹ complexe |
| NH₂ | ∼ 3442 cm⁻¹, ∼ 3355 cm⁻¹ |
| C=O | 1721 cm⁻¹ |
| Aromatique | 1614 cm⁻¹ |
| Hétéroaromatique | 1592 cm⁻¹ |
| NH₂ | 1565-1543-1517 cm⁻¹ |

### EXEMPLE 6 : 2-butyl α-hydroxy α-méthyl 4-(méthylthio) 1-[(2'-((((phénylméthoxy) carbonyl) amino) sulfonyl) (1,1'-biphényl) 4-yl) méthyl] 1H-imidazole 5-acétate d'éthyle

A une solution de 265 mg du produit de l'exemple 1, dans 4 cm³ de diméthoxy éthane on ajoute 138 mg de K₂CO₃ et 0,08 cm³ de chloroformiate de benzyle. On chauffe 30 mn au reflux, dilue à l'eau, extait à l'AcOEt et purifie sur silice avec pour éluant CHCl₃-MeOH (95-5). On récupère 180 mg de produit attendu.

### EXEMPLE 7 : Acide 2-butyl α-hydroxy α-méthyl 4-(méthylthio) 1-[(2'-((((phénylméthoxy) carbonyl) amino) sulfonyl) (1,1'-biphényl) 4-yl) méthyl) 1H-imidazole 5-acétique

On introduit 311 mg du produit de l'exemple 6, dans 10 cm³ de MeOH, ajoute 10 cm³ de NaOH 2N et agite environ 2 heures à température ambiante. On extrait à l'éther, la phase aqueuse puis l'acidifie à pH 1 par addition d'HClc et extrait par CHCl₃ à 20 % de MeOH, puis évapore. On redissout dans 10 cm³ de soude 2N et 15 cm³ d'eau, filtre, acidifie par addition d'HClc, filtre, lave à l'eau et à l'essence G et sèche. On obtient 216 mg de produit attendu. F = 140-150°C.

| Spectre IR : Nujol | |
|---|---|
| Absorption complexe région OH/NH | |
| C=O | 1744 cm⁻¹ complexe |
| Aromatique | 1620 cm⁻¹ |
| Hétéroaromatique | 1591 cm⁻¹ |
| Amide | 1500 cm⁻¹ |

| Microanalyse concordance pour C₃₂H₃₅N₃O₇S₂ = 637, 78 | | | | | |
|---|---|---|---|---|---|
| | C | H | N | S | O |
| % calculé | 60,26 | 5,53 | 6,59 | 10,05 | 17,56 |
| % trouvé | 59,95 | 5,3 | 6,6 | 10,4 | |

### EXEMPLE 8 : 2-butyl α-hydroxy α-méthyl 4-(méthylthio) 1-[(2'-((((propylamino) carbonyl) amino) sulfonyl) (1,1'-biphényl) 4-yl) méthyl] 1H-imidazole 5-acétate d'éthyle

On introduit 1,3 g du produit de l'exemple 1 dans 40 cm³ d'acétone et ajoute 680 mg de carbonate de potassium. On chauffe au reflux, ajoute 0,25 cm³ d'isocyanate de propyle. Et on poursuit le reflux pendant environ 2 heures, refroidit, filtre, lave à l'acétone et chasse le solvant. On purifie sur silice avec pour éluant CHCl₃-MeOH (98-2). On empâte à l'éther iso, filtre et sèche à température ambiante. On obtient 170 mg de produit attendu.

| Spectre IR : CHCl₃ | |
|---|---|
| OH | 3510 cm⁻¹ complexe |
| =C-NH | 3403 cm⁻¹ + associé |
| >=O | 1707 cm⁻¹ |
| Amide II | 1615 cm⁻¹ |
| Aromatique | 1603 cm⁻¹ |
| Hétéroaromatique | 1539 cm⁻¹ (F). |

### EXEMPLE 9 : Acide 2-butyl α-hydroxy α-méthyl 4-(méthylthio) 1-[(2'-((((propylamino) carbonyl) amino) sulfonyl) (1,1'-biphényl) 4-yl) méthyl] 1H-imidazole 5-acétique

A une solution de 582 mg du produit de l'exemple 8 dans 10 cm³ de MeOH on ajoute 5 cm³ de NaOH 2N. On agite à température ambiante pendant environ 5 heures. On chasse le MeOH sous vide, dilue à l'eau et extrait une fraction neutre à l'AcOEt. On filtre la phase aqueuse, acidifie par addition de HClc et extrait par CHCl₃ à 20 % de MeOH puis évapore. On empâte à l'éther iso sous agitation, filtre et sèche à température ambiante. Pour purifier, on dissout 420 mg de produit obtenu dans 50 cm³ de soude N sous agitation. On extrait une fraction neutre à l'éther, filtre la phase aqueuse et acidifie par addition lente d'HClc sous agitation. On agite encore environ 30 mn, filtre, lave à l'eau et à l'essence G et sèche. On obtient 170 mg de produit attendu.

| Spectre IR : | |
|---|---|
| Région OH très complexe | |
| =C-NH | 3400 cm⁻¹ |
| C=O | 1753 - 1705 - 1632 cm⁻¹ |
| Aromatique | 1592 cm⁻¹ |
| Hétéroaromatique | 1551 cm⁻¹ (F). |
| Amide II | 1504 cm⁻¹ |

| Spectre UV : | |
|---|---|
| Dans EtOH pour M = 588,75 | |
| Max. 268 nm | ε = 3100 |
| Max. 276 nm | ε = 2800 |
| Infl. 215, 236 nm | |

### EXEMPLE 10 : 2-butyl α-hydroxy α-méthyl 4-(méthylthio) 1-[(2'-((((phénylméthyl) amino) carbonyl) amino) sulfonyl) (1,1'-biphényl) 4-yl) méthyl] 1H-imidazole 5-acétate d'éthyle

On introduit 500 mg du produit de l'exemple 1 dans 15 cm³ d'acétone et ajoute 260 mg de carbonate de potassium. On chauffe au reflux et introduit 0,13 cm³ d'isocyanate de benzyle. Puis on poursuit le reflux pendant encore 1 heure. On filtre, chasse le solvant et purifie sur silice avec pour éluant CHCl₃-MeOH (98-2). On récupère 590 mg de produit attendu.

### EXEMPLE 11 : Acide 2-butyl α-hydroxy α-méthyl 4-(méthylthio) 1-[(2'-((((phénylméthyl) amino) carbonyl) amino) sulfonyl) (1,1'-biphényl) 4-yl) méthyl) 1H-imidazole 5-acétique

A une solution de 590 mg du produit de l'exemple 10 dans 10 cm³ de méthanol on ajoute 5 cm³ de soude 2N et agite à température ambiante pendant environ 1 heure. On glace, dilue à l'eau, acidifie par HClc et extrait par CHCl₃ à 20 % de MeOH. Pour purifier, on dissout le produit obtenu dans 20 cm³ de NaOH 2N et 30 cm³ d'eau, extrait à l'éther, filtre la solution aqueuse et acidifie sous agitation par addition d'HCl concentré. On filtre et lave à l'eau, puis sèche. On obtient 444 mg de produit attendu. Fk = 158-160°C.

| Microanalyse concordance pour C₃₂H₃₆N₄O₆S₂ = 636,79 | | | | |
|---|---|---|---|---|
| | C | H | N | S |
| % calculé | 60,36 | 5,70 | 8,80 | 10,07 |
| % trouvé | 60,2 | 5,5 | 8,6 | 10,2 |

### EXEMPLE 12 : 2-butyl 1-[(2'-(((((cyclohexylméthyl) amino) carbonyl) amino) sulfonyl) (1,1'-biphényl) 4-yl) méthyl] α-hydroxy α-méthyl 4-(méthylthio) 1H-imidazole 5-acétate d'éthyle

On introduit 178 mg du produit de l'exemple 1 dans 10 cm³ d'acétone et ajoute 46 mg de carbonate de potassium. On chauffe au reflux puis on introduit 47 mg de cyclohexyméthylisocyanate. On chauffe environ 1 heure au reflux, filtre l'insoluble et chasse le solvant. On purifie sur silice avec pour éluant AcOEt-hexane (60-40), puis CHCl₃-MeOH (95-5).

On récupère 200 mg de produit attendu.

### EXEMPLE 13 : Acide 2-butyl 1-[(2'-(((((cyclohexylméthyl) amino) carbonyl) amino) sulfonyl) (1,1'-biphényl) 4-yl) méthyl] α-hydroxy α-méthyl 4-(méthylthio) 1H-imidazole 5-acétique

On introduit 200 mg du produit de l'exemple 12 dans 2 cm³ d'éthanol ajoute 0,2 cm³ de KOH 6N et agite à température ambiante pendant environ 48 heures. On dilue à l'eau et acidifie par addition d'HCl concentré. On filtre, lave à l'eau et sèche. Pour purifier, on dissout dans 60 cm³ de NaOH 2N et 9 cm³ d'eau. On extrait à l'éther, filtre la phase aqueuse, acidifie par HCl concentré, filtre, lave à l'eau et sèche à température ambiante. On obtient 129 mg de produit attendu.

| Microanalyse pour C₃₂H₄₂N₄O₆S₂ = 642, 84 | | | | |
|---|---|---|---|---|
| | C | H | N | S |
| % calculé | 59,79 | 6,59 | 8,72 | 9,98 |
| % trouvé | 59,3 | 6,5 | 8,7 | 9,9 |

### EXEMPLE 20 : α-hydroxy 4-(méthylthio) 1-[(2'-(((((phénylméthyl) amino) carbonyl) amino) sulfonyl) (1,1'-biphényl) 4-yl) méthyl] 2-propyl 1H-imidazole 5-acétate d'éthyle

### Stade A : 1-[(2'-(((éthoxycarbonyl) amino) sulfonyl) (1,1'-biphényl) 4-yl) méthyl] 4-(méthylthio) α-oxo 2-propyl 1H-imidazole 5-acétate d'éthyle

On introduit 3,3 g du produit obtenu à la préparation 3, dans 60 cm³ de diméthoxyéthanol, à laquelle on ajoute successivement 1,81 g de carbonate de potassium puis 1,25 cm³ de chloroformiate d'éthyle. La solution est alors portée au reflux pendant environ 5 heures puis filtre, lave par de l'acétate d'éthyle et la solution organique est amenée à sec. On reprend par du CH₂Cl₂, lave abondamment à l'eau puis sèche. On purifie par chromatographie sur silice avec pour éluant : CH₂Cl₂-méthanol 96-4 et obtient 1,5 g de produit attendu.

| Spectre IR : CHCl₃ | |
|---|---|
| =C-NH | 3384 cm⁻¹ |
| C=O | 1739 - 1626 cm⁻¹ |
| Aromatique | 1564 cm⁻¹ |
| Hétéroatome | 1516 cm⁻¹ |
| Amide II | 1480 cm⁻¹ |

### Stade B : 1-[(2'-(((éthoxycarbonyl) amino) sulfonyl) (1,1'-biphényl) 4-yl) méthyl] α-hydroxy 4-(méthylthio) 2-propyl 1H-imidazole 5-acétate d'éthyle

On introduit 1,3 g du produit obtenu au stade A ci-dessus dans 50 cm³ d'éthanol, refroidit à environ -20°C/-25°C et ajoute 90 mg de borohydrure de sodium. L'agitation est maintenue à environ -20°C pendant environ 15 mn. On évapore l'éthanol puis reprend par 100 cm³ d'eau glacée et ajoute 50 cm³ d'acétate d'éthyle, puis évapore la phase organique, essore, lave abondamment puis sèche. On obtient 1,05 g de produit attendu. F = 147°C.

| Spectre IR : CHCl₃ | |
|---|---|
| Absence de cétone conjuguée | |
| OH | 3590 - 3508 cm⁻¹ |
| - NH + absorption générale | 3385 cm⁻¹ |
| C=O | 1746 cm⁻¹ |
| Aromatique | 1614 - 1590 cm⁻¹ |
| Hétérocycle | 1560 - 1540 cm⁻¹ |
| Amide | 1538 - 1500 cm⁻¹ |

### Stade C : α-hydroxy 4-(méthylthio) 1-[(2'-(((((phénylméthyl) amino) carbonyl) amino) sulfonyl) (1,1'-biphényl) 4-yl) méthyl] 2-propyl 1H-imidazole 5-acétate d'éthyle

On introduit 1 g du produit obtenu au stade B ci-dessus, dans 30 cm³ de toluène, à laquelle on ajoute 1 cm³ de benzylamine, et porte au reflux pendant environ 30 mn. On sèche puis reprend par de l'eau et acidifie par addition d'hydrogéno phosphate de sodium. On extrait par de l'acétate d'éthyle, la phase organique est lavée à l'eau puis séchée. On purifie sur silice avec pour éluant CH₂Cl₂-éthanol (96-4) et obtient 730 mg de produit attendu.

| Spectre IR : CHCl₃ | |
|---|---|
| Absorption OH | 3500 cm⁻¹ |
| NH | 3370 cm⁻¹ complexe |
| C=O | 1714 cm⁻¹ |
| Système conjugué | 1664 - 1592 cm⁻¹ |
| Aromatique Amide II | 1538 - 1502 cm⁻¹ |

### EXEMPLE 21 : Acide 1-[(2'-(((((phénylméthyl) amino) sulfonyl) (1,1'-biphényl) 4-yl) méthyl] α-hydroxy 4-(méthylthio) 2-propyl 1H-imidazole 5-acétique

On introduit 400 mg du produit de l'exemple 20 dans le mélange éthanol-NaOH (5 cm³-5 cm³) et l'agitation est maintenue environ 3 h à température ambiante. Puis on concentre, reprend par de l'eau et acidifie par addition HCl N, essore, lave abondamment à l'eau puis sèche. Pour purifier, on dissout à chaud dans un mélange acétate d'éthyle 25 cm³ et d'éthanol 25 cm³, filtre, concentre de moitié, puis laisse 1 nuit à température ambiante. Après essorage, lavage du précipité successivement par 20 cm³ d'acétate d'éthyle puis 20 cm' d'éthanol et séchage, on récupère 136 mg de produit attendu.
F = 225°C.

| Spectre IR : CHCl₃ | |
|---|---|
| Absorption OH/NH | |
| C=O | 1672 - 1640 cm⁻¹ |

### EXEMPLE 22 : 1-[(2'-(((((cyclohexylméthyl) amino) carbonyl) amino) sulfonyl) (1,1'-biphényl) 4-yl) méthyl] 4-(méthylthio) 2-propyl 1H-imidazole 5-carboxylate d'éthyle

On introduit 400 mg du produit obtenu à la préparation 5 dans 5 ml d'acétone anhydre et 236 mg de carbonate de potassium K₂CO₃. On porte au reflux et ajoute 200 µl d'isocyate de cyclohexylméthyle. Après environ 1 h d'agitation à chaud, la solution est refroidie à température ambiante, hydrolysée par une solution NH₃Cl aqueuse saturée puis extraite au CH₂Cl₂. Après séchage et évaporation, on recristallise dans l'éther après dissolution dans le minimum de CH₂Cl₂, puis filtre, sèche et obtient 420 mg de produit attendu (solide blanc).

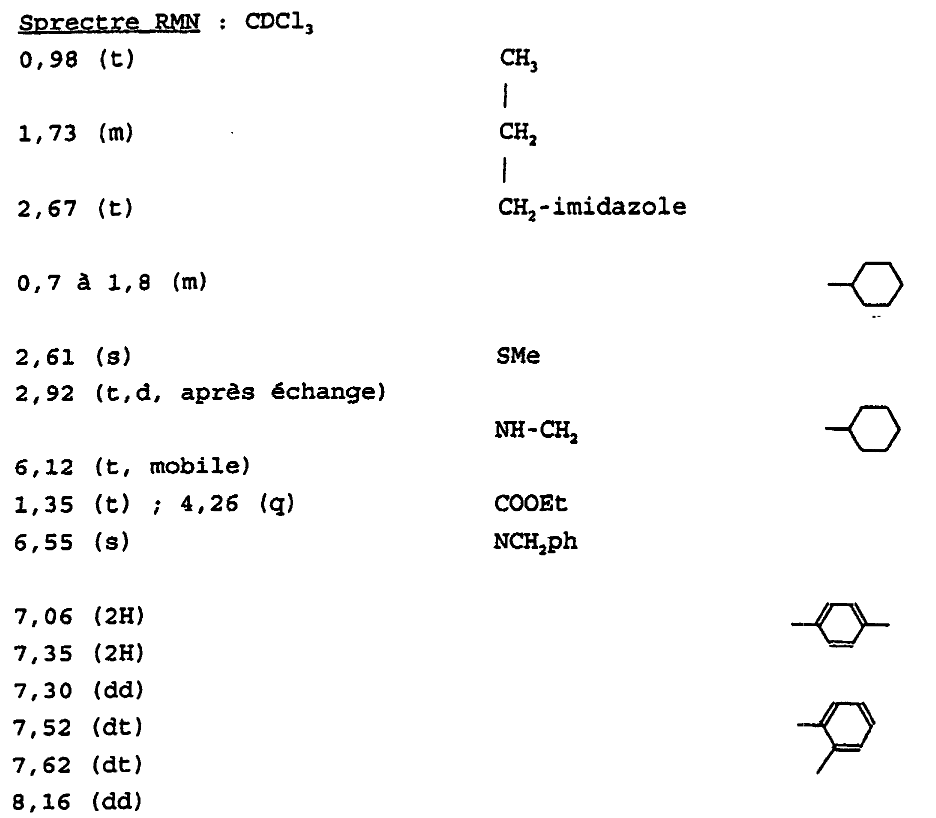

### EXEMPLE 23 : 1-[(2'-(((((cyclohexylméthyl) amino) carbonyl) amino) sulfonyl) (1,1'-biphényl) 4-yl) méthyl] 4-(méthylthio) 2-propyl 1H-imidazole 5-carboxylate de sodium

On introduit 380 mg du produit de l'exemple 22 dans 8 ml d'éthanol et 5 ml de NaOH 2N et laisse sous agitation à température ambiante pendant environ 48 heures. L'éthanol est ensuite évaporé et après ajout de 10 ml d'eau, la phase aqueuse est extraite par de l'acétate d'éthyle, puis filtrée, placée à 0°C et acidifiée lentement jusqu'à pH 2. Après environ 30 mn d'agitatin, le précipité est filtré et séché et on obtient 240 mg de produit attendu.

| Spectre IR : CHCl₃ | |
|---|---|
| Acide d'après région OH | |
| =C-NH | 3390 cm⁻¹ |
| >=O | 1705 - 1680 cm⁻¹ |
| Aromatique | 1606 cm⁻¹ |
| Hétéroatome | 1545 cm⁻¹ |
| Amide II | 1517 cm⁻¹ |

### EXEMPLE 24 : 4-(méthylthio) 1-[(2'-(((((phénylméthyl) amino) carbonyl) amino) sulfonyl) (1,1'-biphényl) 4-yl) méthyl] 2-propyl 1H-imidazole 5-carboxylate d'éthyle

On introduit 2 g du produit obtenu à la préparation 5, dissous dans 25 ml d'acétone anhydre et 1,2 g de carbonate de potassium. On porte au reflux et ajoute 740 µl de benzylisocyanate. Après environ 2 h d'agitation, on refroidit à température ambiante, hydrolyse par une solution aqueuse saturée de NH₄Cl puis extrait au chlorure de méthylène. On sèche, recristallise dans l'éther, filtre et obtient 1,9 g de produit attendu.

### EXEMPLE 25 : acide 4-(méthylthio) 1-[(2'-(((((phénylméthyl) amino) carbonyl) amino) sulfonyl) (1,1'-biphényl) 4-yl) méthyl] 2-propyl 1H-imidazole 5-carboxylique

On introduit 1,8 g du produit obtenu à l'exemple 24, dans 10 ml d'éthanol et 10 ml de soude 2N puis laisse sous agitation à température ambiante pendant environ 36 heures. L'éthanol est ensuite évaporé et, après ajoute de 25 ml d'eau, la phase aqueuse est extraite avec de l'éther, puis filtrée, placée à 0°C et acidifiée lentement jusqu'à pH 1,5 avec HCl 1N. On filtre et sèche et obtient 1,4 g de produit attendu.

| Spectre IR : CHCl₃ | |
|---|---|
| Acide d'après la région OH | |
| =C-NH | 3480 cm⁻¹ |
| >=O | 1706 - 1690 cm⁻¹ complexe |
| Aromatique | 1539 cm⁻¹ |
| Hétéroaromatique | 1521 cm⁻¹ |
| Amide II | 1500 cm⁻¹ |

### EXEMPLE 31 : α-butyl α-hydroxy 4- (méthylthio) 1- [(2'-(((((phénylméthyl) amino) carbonyl) amino) sulfonyl) (1,1'-biphényl) 4-yl) méthyl] 2-propyl 1H-imidazole 5-acétate d'éthyle

### Stade A : 4-(méthylthio) alpha-oxo 1-((2'-(((((phénylméthyl) amino) carbonyl) amino) sulfonyl) (1,1'-biphényl) 4-yl) méthyl) 2-propyl 1H-imidazole 5-acétate d'éthyle

On introduit 3,6 g de produit obtenu à la préparation 3 dans 50 cm³ d'acétone puis en une fois 2 g de carbonate de potassium. Le milieu est alors porté au reflux et on y ajoute goutte à goutte 1 cm³ d'isocyanate de benzyle. Le reflux est maintenu durant 2 h. Puis on verse sur 500 cm³ d'eau glacée, acidifie le milieu par addition d'acide chlorhydrique N, essore, lave abondamment à l'eau, sèche à 50°C. On purifie par empâtage dans 50 cm³ d'éther isopropylique et obtient 4,2 g de produit dont 500 mg sont purifiés par recristallisation dans 25 cm³ d'éthanol, après essorage et séchage, on obtient 300 mg de produit attendu. F = 188°C.

| Spectre IR : CHCl₃ | |
|---|---|
| =C-NH | 3375 cm⁻¹ |
| C=O | 1714 - 1621 cm⁻¹ |
| Système conjugué + | 1537 cm⁻¹ |
| Aromatique + Amide II + | 1495 cm⁻¹ |

### Stade B : α-butyl α-hydroxy 4-(méthylthio) 1-[(2'-(((((phénylméthyl) amino) carbonyl) amino) sulfonyl) (1,1'-biphényl) 4-yl) méthyl] 2-propyl 1H-imidazole 5-acétate d'éthyle

On introduit 750 mg du produit obtenu au stade A ci-dessus, dans 50 cm³ de THF anhydre, refroidit à 0°C et ajoute goutte à goutte 2,8 cm³ de chlorure de n-butyl magnésium, l'agitation est maintenue à 0°C pendant 30 mn puis à température ambiante pendant 1 h. Le milieu réactionnel est acidifié par addition d'acide chlorhydique 0,1 N, puis on extrait par de l'acétate d'éthyle. La phase organique est abondamment lavée par de l'eau puis séchée. On récupère une résine jaune. On purifie par 2 chromatographies successives sur silice avec pour éluant CH₂Cl₂ + 2,5 % méthanol et obtient 300 mg de produit attendu.

| Spectre IR : CHCl₃ | |
|---|---|
| OH | 3520 cm⁻¹ |
| NH | 3408 cm⁻¹ |
| C=O | 1714 cm⁻¹ |
| Système conjugué | 1604 cm⁻¹ |
| Aromatique | 1532 cm⁻¹ (complexe, F) |
| Amide II | 1500 cm⁻¹ |

### EXEMPLE 32 : α-butyl α-hydroxy 4-(méthylthio) 1-[(2'-(((((phénylméthyl) amino) carbonyl) amino) sulfonyl) (1,1'-biphényl) 4-yl) méthyl] 2-propyl 1H-imidazole 5-acétate de sodium

On introduit 260 mg du produit obtenu à l'exemple 31, dans 5 cm³ de soude, 20 cm³ d'éthanol, l'agitation est maintenue pendant 4 h à température ambiante. Le milieu réactionnel est acidifié par addition d'HCl 2N, après extraction par de l'acétate d'éthyle, lavage à l'eau de la phase organique, puis séchage, on purifie par chromatographie sur silice avec pour éluant CH₂Cl₂-méthanol. On purifie à nouveau par HPLc sur silice avec pour éluant H₂O-méthanol (60-40) et obtient 90 mg de produit attendu. F = 210°C.

| Spectre IR : Nujol | |
|---|---|
| Absorption complexe région OH/NH | |
| C=O | 1610 cm⁻¹ (F) |
| Aromatique | 1520 cm⁻¹ |
| Hétéroatome Amide II | 1500 cm⁻¹ |

### EXEMPLE 34 : Acide α-hydroxy 4-(méthylthio) α-phényl 1-((2'-(((((phénylméthyl) amino) carbonyl) amino) sulfonyl) (1,1'-biphényl) 4-yl) méthyl) 2-propyl 1H-imidazole 5-acétique

On dissout 250 mg de produit obtenu au stade A de l'exemple 31 dans 5 cm³ de THF anhydre. On ajoute goutte à goutte 430 µl de PhMgBr 3M dans. l'éther. Après une heure à température ambiante, on hydrolyse avec HCl 2N, extrait à l'ACOEt, sèche et évapore à sec.

Le brut obtenu est dissous dans un mélange.de 5 cm³ d'éthanol et 5 cm³ NaOH 2N. Après une nuit à température ambiante, on acidifie avec HCl 1N, on filtre, lave le précipité obtenu à l'eau puis avec 5 cm³ d'ACOEt et 5 cm³ d'éther isopropylique. On obtient 67 mg d'un solide blanc. F = 170°C.

### EXEMPLE 87 de composition pharmaceutique.

On a préparé des comprimés répondant à la formule suivante :

| | |
|---|---|
| Produit (I) | 50 mg |
| Excipient pour un comprimé terminé à (détail de l'excipient : lactose, talc, amidon, stéarate de magnésium). | 200 mg |

### RESULTATS PHARMACOLOGIQUES :

### 1 - Test sur le récepteur AT₁ de l'angiotensine II

On utilise une préparation membranaire fraîche obtenue à partir de foie de rat. Le tissu est broyé au polytron dans un tampon Tris 50 mM pH 7,4, le broyage est suivi de 3 centrifugations à 30 000 g 15' avec reprises intermédiaires des culots dans le tampon Tris pH 7,4.

Les derniers culots sont remis en suspension dans un tampon d'incubation (Tris 20 mM, NaCl 135 mM, KCl 10 mM, glucose 5 mM, MgCl₂ 135 mM, PMSF 0,3 mM, bacitracine 0,1 mM, lysozyme 0,1 %).

On répartit des fractions aliquotées de 1 ml dans des tubes en verre et ajoute de la ¹²⁵ I angiotensine II (25 000 DPM/tube) et le produit à étudier. (Le produit est d'abord testé à 3 x 10⁻⁵M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %).

La liaison non spécifique est déterminée par addition du produit de l'exemple 94 du brevet européen 0253310, à 10⁻⁵M (en triple). On incube à 25°C pendant 150 minutes, remet au bain-marie à 0°C, 5 minutes, filtre sous vide, rince au tampon Tris pH 7,4 et compte la radioactivité en présence de scintillant solide.

Le résultat est exprimé directement en concentration inhibitrice 50 % (CI₅₀), c'est-à-dire en concentration de produit étudié, exprimée en nM, nécessaire pour déplacer 50 % de la radioactivité spécifique fixée sur le récepteur étudié.

### Résultats :

| Produit de l'exemple | Récepteur AT₁ CI₅₀ en nanomoles |
|---|---|
| 7 | 0, 7 |
| 9 | 3,4 |
| 11 | 24 |
| 13 | 6,3 |
| 21 | 2,5 |
| 23 | 0,2 |
| 25 | 0,2 |
| 32 | 0,5 |
| 34 | 0, 7 |

### 2) Test sur le récepteur AT₂ de l'angiotensine II

On utilise une préparation membranaire fraîche obtenue à partir d'utérus de lapine, prétraitées 4 jours auparavent par 50 µg d'estradiol administré par voie percutanée. Le tissu est broyé au polytron dans un tampon Tris 50 mM, pH 7,4 et le broyage est suivi de 3 centrifugations à 30 000 g 15 minutes, avec reprises intermédiaires des culots dans le tampon Tris pH 7,4.

Les derniers culots sont remis en suspension dans un tampon d'incubation (Tris 20 mM, NaCl 135 mM, KCl 10 mM, glucose 5 mM, MgCl₂, 6H₂O 10 mM, PMSF 0,3 mM, bacitracine 0,1 mM, lysozyme 0,1 %, pH 7,4).

L'homogénat obtenu est mis à préincuber 20 minutes à 25°C en présence de dithiothreitol 10 mM, puis ramené à 0°-4°C.

On répartit des fractions aliquotées de 1 ml dans des tubes en verre et ajoute de la ¹²⁵I angiotensine II (25 000 DPM/tube) et le produit à étudier. Le produit est d'abord testé à 3.10⁻⁵M en triple. Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.

La liaison spécifique est déterminée par addition de EXP 655 (= PD 123-177 de Warner-Lamber) à 10⁻⁵M en triple. On incube à 25°C pendant 150 minutes, remet au bain-marie à 0°C, 5 minutes, filtre sous vide, rince au tampon Tris pH 7,4 et compte la radioactivité en présence de scintillant solide.

Le résultat est exprimé directement en concentration inhibitrice 50 % (CI₅₀), c'est-à-dire en concentration de produit étudié, exprimée en nM, nécessaire pour déplacer 50 % de la radioactivité spécifique fixée sur le récepteur étudié.

### Résultats :

| Produit de l'exemple | Récepteur AT₂ CI₅₀ en nanomoles |
|---|---|
| 7 | 5,2 |
| 9 | 8, 5 |
| 11 | 1, 6 |
| 13 | 1, 9 |
| 21 | 6,5 |
| 23 | 2,0 |
| 25 | 5,9 |
| 32 | 0,8 |
| 34 | 2,1 |

### 3 - Test d'activité antagoniste de l'angiotensine II chez le rat démédullé

Des rats mâles Sprague-Dawley (250 à 350 g) sont anesthésiés par une injection intra-péritonéale de pentobarbital sodique (60 mg/kg). La pression artérielle diastolique est enregistrée grâce à un cathéter (PE50) hépariné introduit dans la carotide gauche de l'animal, et relié à un calculateur de pression (Gould, Pressure Processor) par l'intermédiaire d'un capteur de pression Gould.

Un cathéter est introduit dans la jugulaire droite de l'animal afin de permettre l'injection des molécules à étudier.

L'animal est placé sous respiration assistée. Une section bilatérale des nerfs vagues est effectuée. Le rat est alors démédullé.

Après une période de stabilisation suffisante, l'étude de l'antagonisme des molécules vis-à-vis de l'angiotensine II (Hypertensine, CIBA) est abordée de la façon suivante :
1 - Trois injections consécutives d'angiotensine II (0,75 microgrammes/kg) espacées de 15 minutes permettent d'obtenir une réponse pressive reproductible et stable.
2 - Tout en gardant une périodicité de 15 minutes pour l'administration d'angiotensine II, les molécules (0,01 à 10 mg/kg) sont injectées 5 minutes avant l'angiotensine II.
   Les effets presseurs de l'angiotensine II en présence de l'antagoniste sont exprimés en pourcentage des effets presseurs de l'angiotensine II administrée seule. La dose inhibitrice à 50 % (DI₅₀) de l'effet étudié est ainsi déterminée.
   Chaque animal est considéré comme son propre témoin.

### Résultats :

| Produit de l'exemple | DI₅₀ en mg/kg | |
|---|---|---|
| | IV | PO |
| 7 | 0,6 | - |
| 23 | 0,3 | 1,54 |
| 25 | 0,22 | 1,45 |
| 32 | 0,46 | - |

## Revendications

1. Utilisation des produits de formule (I) 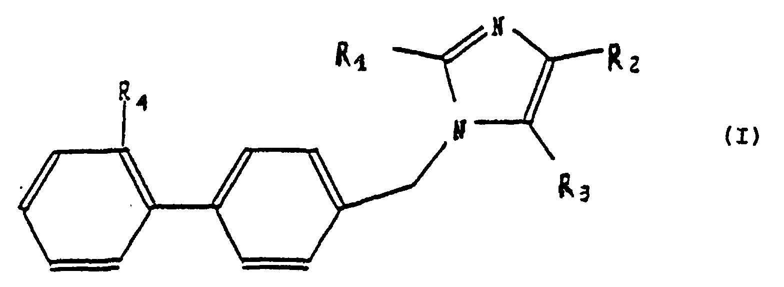 répondant aux formules suivantes :
- Acide 2-butyl α-hydroxy α-méthyl 4-(méthylthio) 1-[(2'-((((phénylméthoxy) carbonyl) amino) sulfonyl) (1,1'-biphényl) 4-yl) méthyl] 1H-imidazole 5-acétique,
- Acide 2-butyl α-hydroxy α-méthyl 4-(méthylthio) 1-[(2'-((((propylamino) carbonyl) amino) sulfonyl) (1,1'-biphényl) 4-yl) méthyl] 1H-imidazole 5-acétique,
- Acide 2-butyl α-hydroxy α-méthyl 4-(méthylthio) 1-[(2'-((((phénylméthyl) amino) carbonyl) amino) sulfonyl) (1,1'-biphényl) 4-yl) méthyl] 1H-imidazole 5-acétique,
- Acide 2-butyl 1-[(2'-(((((cyclohexylméthyl) amino) carbonyl) amino) sulfonyl) (1,1'-biphényl) 4-yl) méthyl] α-hydroxy α-méthyl 4-(méthylthio) 1H-imidazole 5-acétique,
- Acide 1-[(2'-(((((phénylméthyl) amino) sulfonyl) (1,1'-biphényl) 4-yl) méthyl] α-hydroxy 4-(méthylthio) 2-propyl 1H-imidazole 5-acétique,
- 1-[(2'-(((((cyclohexylméthyl) amino) carbonyl) amino) sulfonyl) (1,1'-biphényl) 4-yl) méthyl] 4-(méthylthio) 2-propyl 1H-imidazole 5-carboxylate de sodium,
- Acide 4-(méthylthio) 1-[(2'-(((((phénylméthyl) amino) carbonyl) amino) sulfonyl) (1,1'-biphényl) 4-yl) méthyl] 2-propyl 1H-imidazole 5-carboxylique,
- α-butyl α-hydroxy 4-(méthylthio) 1-[(2'-(((((phénylméthyl) amino) carbonyl) amino) sulfonyl) (1,1'-biphényl) 4-yl) méthyl] 2-propyl 1H-imidazole 5-acétate de sodium,
- Acide α-hydroxy 4-(méthylthio) α-phényl 1-((2'-(((((phénylméthyl) amino) carbonyl) amino) sulfonyl) (1,1'-biphényl) 4-yl) méthyl) 2-propyl 1H-imidazole 5-acétique,
lesdits produits de formule (I) étant sous toutes les formes isomères racémiques, énantiomères et diastéréoisomères possibles ainsi que les sels d'addition avec les acides minéraux et organiques ou avec les bases minérales et organiques desdits produits de formule (I) pour la préparation de compositions pharmaceutiques destinées au traitement d'affections résultant d'une stimulation anormale aux récepteurs AT₁ et AT₂ de l'Angiotensine II.

2. Utilisation des produits tels que définis à la revendication 1, pour la préparation de compositions pharmaceutiques destinées au traitement de l'hypertension artérielle, de l'insuffisance cardiaque et des resténoses post-angioplastie.

3. Utilisation des produits tels que définis à la revendication 1, pour la préparation de compositions pharmaceutiques destinées au traitement de l'insuffisance rénale.

4. Utilisation des produits tels que définis à la revendication 1, pour la préparation de compositions pharmaceutiques destinées au traitement et à la prévention des désordres cardiovasculaires associés au diabète.

5. Les produits répondant aux formules suivantes :
- Acide 2-butyl α-hydroxy α-méthyl 4-(méthylthio) 1-[(2'-((((phénylméthoxy) carbonyl) amino) sulfonyl) (1,1'-biphényl) 4-yl) méthyl] 1H-imidazole 5-acétique,
- Acide 2-butyl α-hydroxy α-méthyl 4-(méthylthio) 1-[(2'-((((propylamino) carbonyl) amino) sulfonyl) (1,1'-biphényl) 4-yl) méthyl] 1H-imidazole 5-acétique,
- Acide 2-butyl α-hydroxy α-méthyl 4-(méthylthio) 1-[(2'-((((phénylméthyl) amino) carbonyl) amino) sulfonyl) (1,1'-biphényl) 4-yl) méthyl] 1H-imidazole 5-acétique,
- Acide 2-butyl 1-[(2'-(((((cyclohexylméthyl) amino) carbonyl) amino) sulfonyl) (1,1'-biphényl) 4-yl) méthyl] α-hydroxy α-méthyl 4-(méthylthio) 1H-imidazole 5-acétique,
- Acide 1-[(2'-(((((phénylméthyl) amino) sulfonyl) (1,1'-biphényl) 4-yl) méthyl] α-hydroxy 4-(méthylthio) 2-propyl 1H-imidazole 5-acétique,
- 1-[(2'-(((((cyclohexylméthyl) amino) carbonyl) amino) sulfonyl) (1,1'-biphényl) 4-yl) méthyl] 4-(méthylthio) 2-propyl 1H-imidazole 5-carboxylate de sodium,
- Acide 4-(méthylthio) 1-[(2'-(((((phénylméthyl) amino) carbonyl) amino) sulfonyl) (1,1'-biphényl) 4-yl) méthyl] 2-propyl 1H-imidazole 5-carboxylique,
- α-butyl α-hydroxy 4-(méthylthio) 1-[(2'-(((((phénylméthyl) amino) carbonyl) amino) sulfonyl) (1,1'-biphényl) 4-yl) méthyl] 2-propyl 1H-imidazole 5-acétate de sodium,
- Acide α-hydroxy 4-(méthylthio) α-phényl 1-((2'-(((((phénylméthyl) amino) carbonyl) amino) sulfonyl) (1,1'-biphényl) 4-yl) méthyl) 2-propyl 1H-imidazole 5-acétique,

6. Procédé de préparation des produits tels que définis à la revendication 1, **caractérisé en ce que** :
soit l'on soumet un composé de formule (II) : 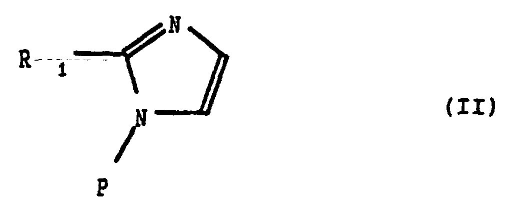 dans laquelle R₁ a la signification indiquée à la revendication 1 et P représente un groupement protecteur de l'atome d'azote,
à une réaction d'halogénation pour obtenir un composé de formule (III) : 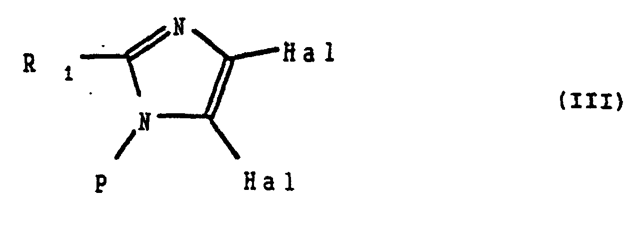 dans laquelle R ₁ et P ont les significations indiquées ci-dessus et Hal représente un atome d'halogène, que l'on soumet à une réaction d'échange halogène-métal sur l'un des atomes d'halogène puis à une réaction avec un composé de formule (IV_{c}) ou (IV_{d}) : 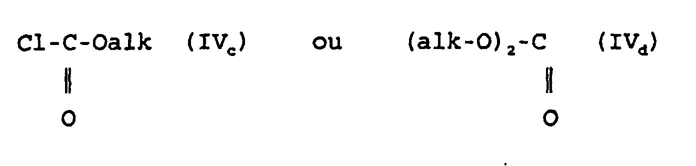 dans lesquelles alk représente un radical alkyle renfermant au plus 4 atomes de carbone,
pour obtenir le composé de formule (V) : 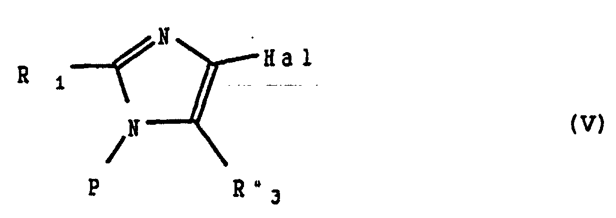 dans laquelle R ₁, P et Hal ont les significations indiquées ci-dessus et R"₃ représente CO-O-alk tel que défini ci-dessus composé de formule (V) que l'on soumet à une réaction d'échange halogène-métal sur l'atome d'halogène puis à une réaction avec un composé de formule (Ivₐ) ou (IV_{b}) :
(-S-R')₂ ( IVa) ou MeSO₂SR' (IV_{b})
dans lesquelles SR' a la signification de R₂ indiquée à la revendication 1 pour obtenir le composé de formule (VII) : 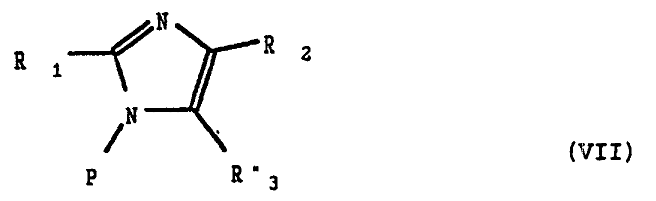 dans laquelle R ₁, R₂, R"₃ et P ont les significations indiquées ci-dessus,
produit de formule (VII) dont on libère la fonction amine bloquée par P tel que défini ci-dessus, puis fait réagir avec un composé de formule (VIII) : 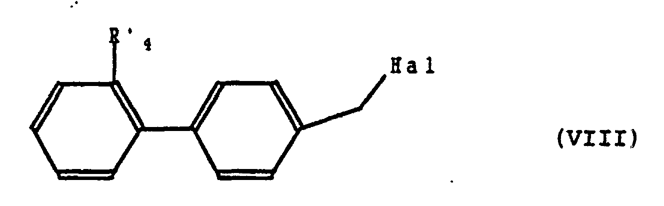 dans laquelle R'₄ a la signification indiquée à la revendication 1 pour R₄, dans laquelle les éventuelles fonctions réactives sont éventuellement protégées par des groupements protecteurs et Hal représente un atome d'halogène pour obtenir un produit de formule (I₁) : 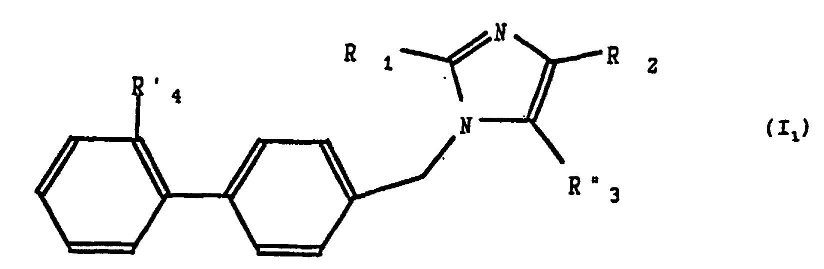 dans laquelle R ₁, R ₂, R"₃ et R'₄ ont les significations indiquées ci-dessus,
soit l'on soumet un composé de formule (IX) : 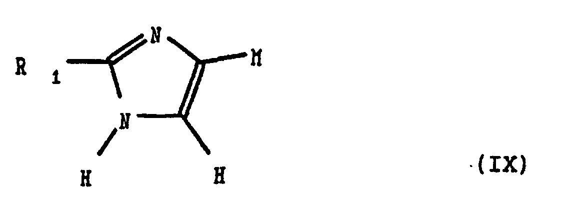 dans laquelle R ₁ a la signification indiquée ci-dessus et M représente un atome d'hydrogène ou le radical R ₂ qui a la signification indiquée ci-dessus,
à une réaction avec le composé de formule (VIII) telle que définie ci-dessus, pour obtenir un produit de formule (X) : 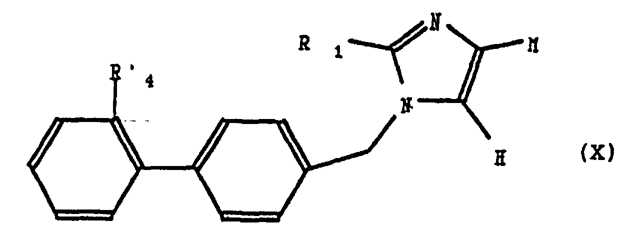 dans laquelle R ₁, M et R'₄ ont les significations indiquées ci-dessus,
produit de formule (X) que lorsque M représente un atome d'hydrogène, l'on soumet à une réaction d'halogénation pour obtenir le produit de formule (XIV) : 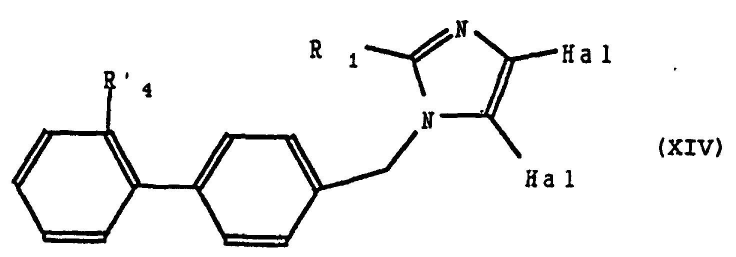 dans laquelle R ₁, R'₄ et Hal ont les significations indiquées ci-dessus, que l'on soumet à une réaction d'échange halogène métal puis à l'action d'un composé de formule (IV_{c}) ou (IV_{d}) telle que définie ci-dessus pour obtenir le produit de formule (XXI): 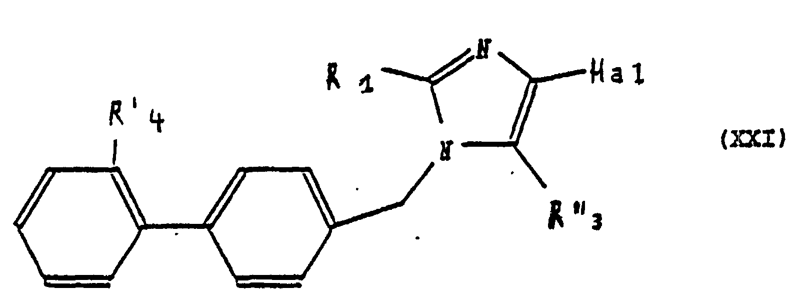 dans laquelle R ₁, R'₄, Hal et R"₃ ont les significations indiquées ci-dessus, produit de formule (XXI) que l'on soumet à une réaction d'échange halogène métal puis à l'action d'un composé de formule (IVₐ) ou (IV_{b}), telle que définie ci-dessus, pour obtenir un produit de formule (I₁) : 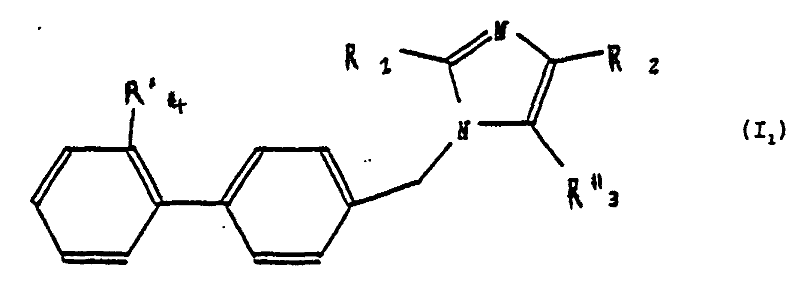 dans laquelle R ₁, R'₄, R ₂ et R"₃ ont les significations indiquées ci-dessus,
soit l'on soumet un composé de formule (XX) : 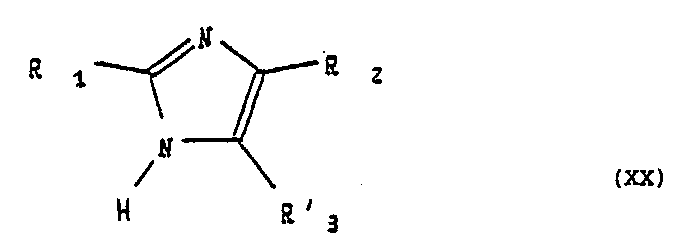 dans laquelle R ₁ et R ₂ ont les significations indiquées ci-dessus et R'₃ a la signification indiquée à la revendication 1 pour R₃ dans laquelle les éventuelles fonctions réactives sont éventuellement protégées par des groupements protecteurs, à une réaction avec le composé de formule (VIII) telle que définie ci-dessus, pour obtenir un produit de formule (I') : 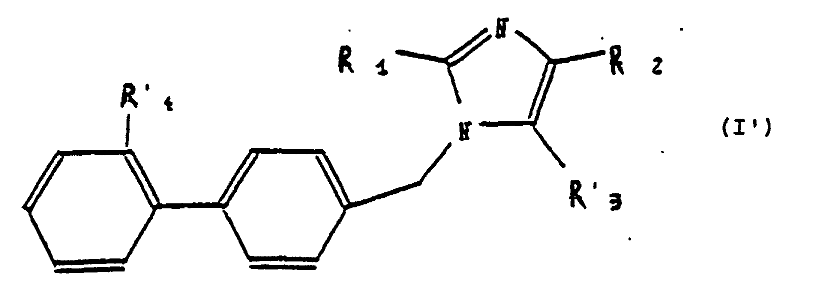 dans laquelle R ₁, R ₂, R'₃ et R'₄ ont les significations indiquées ci-dessus,
produits de formule (I') qui peuvent être des produits de formule (I) et que, pour obtenir des ou d'autres produits de formule (I), l'on peut soumettre, si désiré et si nécessaire, ainsi que les produits de formule (I₁), à l'une ou plusieurs des réactions de transformations suivantes, dans un ordre quelconque :
b) saponification de fonction ester en fonction acide,
d) transformation de fonction cyano en fonction acide,
e) transformation de fonction acide en fonction amide,
g) transformation de fonction alcoxy en fonction hydroxyle,
h) oxydation de fonction alcool en fonction acide
i) transformation d'un radical formyle en radical carbamoyle,
r) transformation d'un carbamate en urée et notamment d'un sulfonylcarbamate en sulfonylurée,
s) élimination des groupements protecteurs que peuvent porter les fonctions réactives protégées,
t) salification par un acide minéral ou organique ou par une base pour obtenir le sel correspondant,
u) dédoublement des formes racémiques en produits dédoublés, lesdits produits tels que définis à la revendication 1 ainsi obtenus étant sous toutes les formes isomères possibles racémiques, énantiomères et diastéréoisomères.

7. A titre de médicaments, les produits tels que définis à de la revendication 5, ces produits étant sous toutes les formes isomères possibles racémiques, énantiomères et diastéréoisomères, ainsi que les sels d'addition avec les acides minéraux et organiques ou avec les bases minérales et organiques pharmaceutiquement acceptables de ces produits tels que définis à la revendication 1.

8. Les compositions pharmaceutiques contenant à titre de principe actif, l'un au moins des médicaments tels que définis à la revendication 7.

## Patentansprüche

1. Use of the products of formula (I): 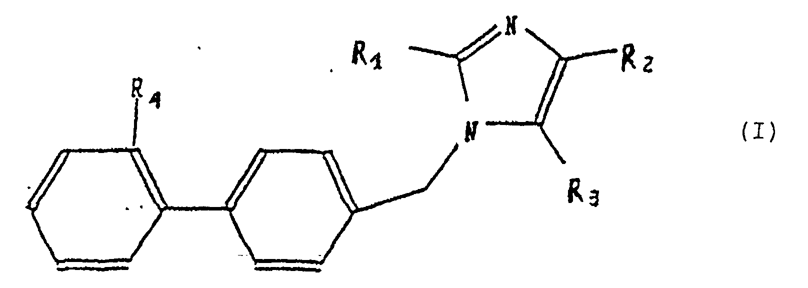
- 2-butyl α-hydroxy α-methyl 4-(methylthio) 1-[(2'-((((phenylmethoxy) carbonyl) amino) sulphonyl) (1,1'biphenyl) 4-yl) methyl] 1H-imidazole 5-acetic acid,
- 2-butyl α-hydroxy α-methyl 4-(methylthio) 1-[(2'-((((propylamino) carbonyl) amino) sulphonyl) (1,1'biphenyl) 4-yl) methyl] 1H-imidazole 5-acetic acid,
- 2-butyl α-hydroxy α-methyl 4-(methylthio) 1-[(2'-((((phenylmethyl) amino) carbonyl) amino) sulphonyl) (1,1'-biphenyl) 4-yl) methyl] 1H-imidazole 5-acetic acid,
- 2-butyl 1-[(2'-(((((cyclohexylmethyl) amino) carbonyl) amino) sulphonyl) (1,1'-biphenyl) 4-yl) methyl] α-hydroxy α-methyl 4-(methylthio) 1H-imidazole 5-acetic acid,
- 1-[(2'-(((((phenylmethyl) amino) sulphonyl) (1,1'biphenyl) 4-yl) methyl] α-hydroxy 4-(methylthio) 2-propyl 1H-imidazole 5-acetic acid,
- sodium 1-[(2'-(((((cyclohexylmethyl) amino) carbonyl) amino) sulphonyl) (1,1'-biphenyl) 4-yl) methyl] 4-(methylthio) 2-propyl 1H-imidazole 5- carboxylate,
- 4-(methylthio) 1-[(2'-(((((phenylmethyl) amino) carbonyl) amino) sulphonyl) (1,1'-biphenyl) 4-yl) methyl] 2-propyl 1H-imidazole 5-carboxylic acid,
- sodium α-butyl α-hydroxy 4-(methylthio) 1-[(2'-(((((phenylmethyl) amino) carbonyl) amino) sulphonyl) (1,1'-biphenyl) 4-yl) methyl] 2-propyl 1H-imidazole 5-acetate,
- α-hydroxy 4-(methylthio) α-phenyl 1-((2'-(((((phenylmethyl) amino) carbonyl) amino) sulphonyl) (1,1'-biphenyl) 4-yl) methyl) 2-propyl 1H-imidazole 5-acetic acid,
said products of formula (I) being in all possible racemic, enantiomeric and diastereoisomeric isomer forms as well as the addition salts with mineral and organic acids or with mineral and organic bases of said products of formula (I) for the preparation of pharmaceutical compositions intended for the treatment of diseases resulting from an abnormal stimulation of the AT₁ and AT₂ receptors of Angiotensin II.

2. Use of the products as defined in claim 1, for the preparation of pharmaceutical compositions intended for the treatment of arterial hypertension, cardiac insufficiency and the post-angioplasty recurrence of stenosis.

3. Use of the products as defined in claim 1, for the preparation of pharmaceutical compositions intended for the treatment of renal insufficiency.

4. Use of the products as defined in claim 1, for the preparation of pharmaceutical compositions intended for the treatment and the prevention of cardiovascular disorders associated with diabetes.

5. Products corresponding to the following formulae:
- 2-butyl α-hydroxy α-methyl 4-(methylthio) 1-[(2'-((((phenylmethoxy) carbonyl) amino) sulphonyl) (1,1'biphenyl) 4-yl) methyl] 1H-imidazole 5-acetic acid,
- 2-butyl α-hydroxy α-methyl 4-(methylthio) 1-[(2'-((((propylamino) carbonyl) amino) sulphonyl) (1,1'biphenyl) 4-yl) methyl] 1H-imidazole 5-acetic acid,
- 2-butyl α-hydroxy α-methyl 4-(methylthio) 1-[(2'-((((phenylmethyl) amino) carbonyl) amino) sulphonyl) (1,1'-biphenyl) 4-yl) methyl] 1H-imidazole 5-acetic acid,
- 2-butyl 1-[(2'-(((((cyclohexylmethyl) amino) carbonyl) amino) sulphonyl) (1,1'-biphenyl) 4-yl) methyl] α-hydroxy α-methyl 4-(methylthio) 1H-imidazole 5-acetic acid,
- 1-[(2'-(((((phenylmethyl) amino) sulphonyl) (1,1'biphenyl) 4-yl) methyl] α-hydroxy 4-(methylthio) 2-propyl 1H-imidazole 5-acetic acid,
- sodium 1-[(2'-(((((cyclohexylmethyl) amino) carbonyl) amino) sulphonyl) (1,1'-biphenyl) 4-yl) methyl] 4-(methylthio) 2-propyl 1H-imidazole 5- carboxylate,
- 4-(methylthio) 1-[(2'-(((((phenylmethyl) amino) carbonyl) amino) sulphonyl) (1,1'-biphenyl) 4-yl) methyl] 2-propyl 1H-imidazole 5-carboxylic acid,
- sodium α-butyl α-hydroxy 4-(methylthio) 1-[(2'-(((((phenylmethyl) amino) carbonyl) amino) sulphonyl) (1,1'-biphenyl) 4-yl) methyl] 2-propyl 1H-imidazole 5-acetate,
- α-hydroxy 4-(methylthio) α-phenyl 1-((2'-(((((phenylmethyl) amino) carbonyl) amino) sulphonyl) (1,1'-biphenyl) 4-yl) methyl) 2-propyl 1H-imidazole 5-acetic acid,

6. Process for the preparation of the products as defined in claim 1, **characterized in that**:
either a compound of formula (II): 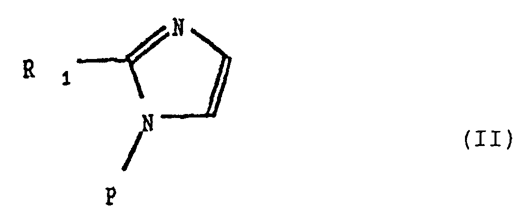 in which R₁ has the meaning indicated in claim 1 and P represents a protective group of the nitrogen atom, is subjected to a halogenation reaction in order to obtain a compound of formula (III): 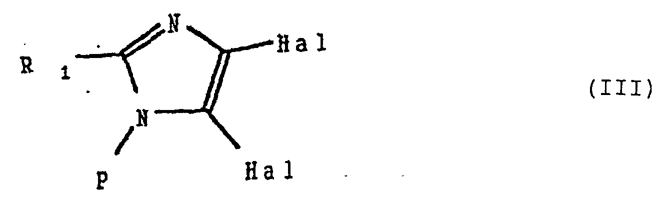 in which R'₁ and P have the meanings indicated above and Hal represents a halogen atom which is subjected to a halogen-metal exchange reaction on one of the halogen atoms, then to a reaction with a compound of formula (IV_{c}) or (IV_{d}): 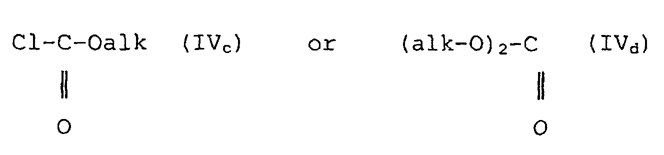 in which alk represents an alkyl radical containing at most 4 carbon atoms,
in order to obtain the compound of formula (V): 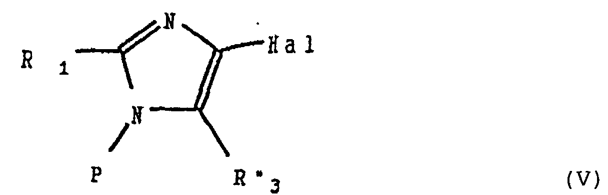 in which R'₁, P and Hal have the meanings indicated above and R"₃ represents CO-O-alk as defined above, which compound of formula (V) is subjected to a halogen-metal exchange reaction on the halogen atom then to a reaction with a compound of formula (Ivₐ) or (IV_{b}) :
(-S-R')₂ (IVa) or MeSO₂SR' (IV_{b})
in which SR' has the meaning of R₂, indicated in claim 1, in order to obtain the compound of formula (VII): 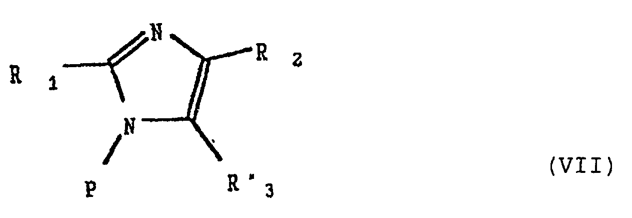 in which R'₁, R₂, R"₃ and P have the meanings indicated above, product of formula (VII) the blocked amine function of which is freed by P as defined above, then reacted with a compound of formula (VIII): 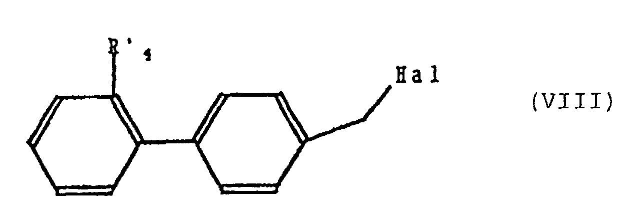 in which R'₄ has the meaning indicated in claim 1 for R₄, in which the optional reactive functions are optionally protected by protective groups and Hal represents a halogen atom in order to obtain a product of formula (I₁): 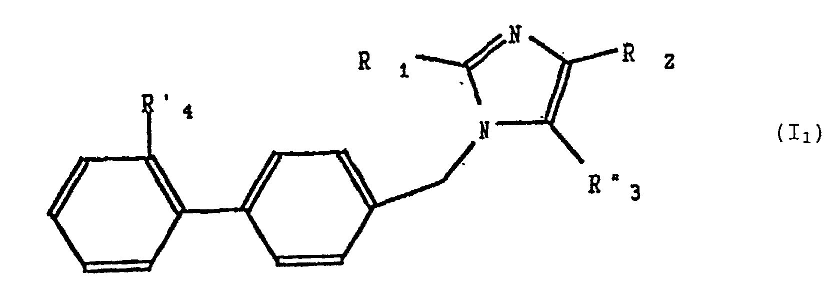 in which R'₁, R₂, R"₃ and R'₄ have the meanings indicated above,
or a compound of formula (IX): 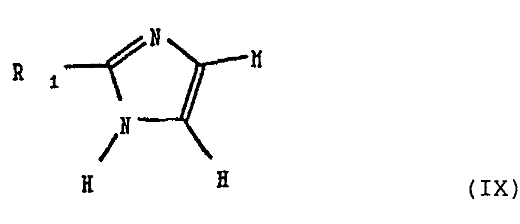 in which R'₁ has the meaning indicated above and M represents a hydrogen atom or the R₂ radical which has the meaning indicated above is subjected to a reaction with the compound of formula (VIII) as defined above, in order to obtain a product of formula (X): 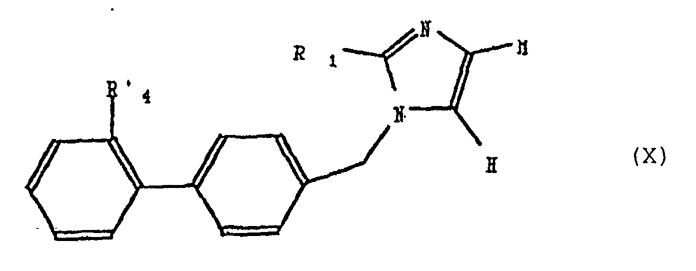 in which R₁, M and R'₄ have the meanings indicated above, product of formula (X) when M represents a hydrogen atom, is subjected to a halogenation reaction in order to obtain the product of formula (XIV) : 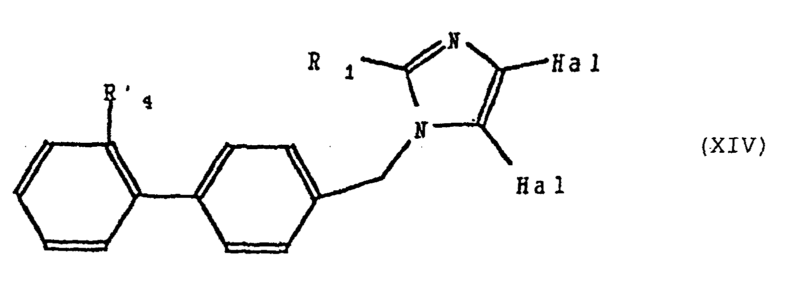 in which R₁, R'₄ and Hal have the meanings indicated above, which is subjected to a halogen-metal exchange reaction then to the action of a compound of formula (IV_{c}) or (IV_{d}) as defined above in order to obtain the product of formula (XXI) : 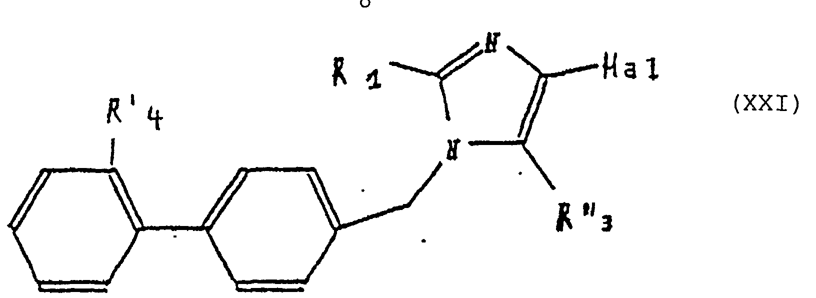 in which R₁, R'₄, Hal and R"₃ have the meanings indicated above, which product of formula (XXI) can be subjected to a halogen-metal exchange reaction then to the action of a compound of formula (IVₐ) or (IV_{b}), as defined above, in order to obtain a product of formula (I₁) : 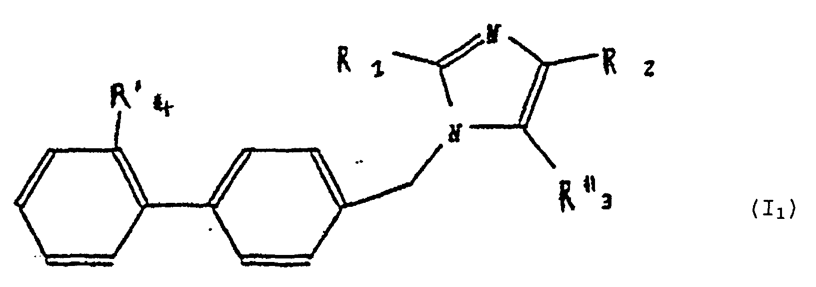 in which R₁, R'₄, R₂ and R"₃ have the meanings indicated above,
or a compound of formula (XX) : 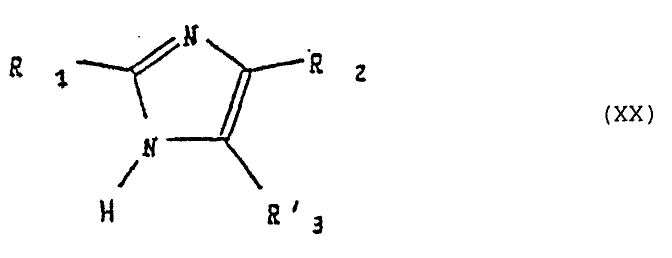 in which R₁ and R₂ have the meanings indicated above and R'₃ has the meaning indicated in claim 1 for R₃ in which the optional reactive functions are optionally protected by protective groups, is subjected to a reaction with the compound of formula (VIII) as defined above, in order to obtain a product of formula (I'): 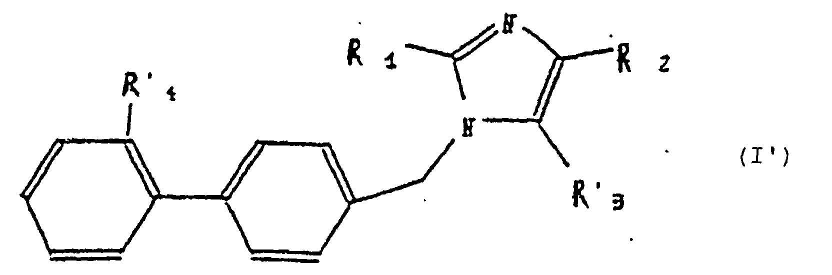 in which R₁, R₂, R'₃ and R'₄ have the meanings indicated above, which products of formula (I') can be products of formula (I) and that, in order to obtain other products of formula (I), can be subjected, if desired and if necessary, as well as the products of formula (I') to one or more of the following conversion reactions, in any order:
b) saponification of the ester function to an acid function,
d) conversion of the cyano function to an acid function,
e) conversion of the acid function to an amide function,
g) conversion of the alkoxy function to a hydroxyl function,
h) oxidation of the alcohol function to an acid function
i) conversion of the formyl radical to a carbamoyl radical,
r) conversion of a carbamate to urea and in particular of a sulphonylcarbamate to sulphonylurea,
s) elimination of the protective groups which can be carried by the protected reactive functions,
t) salification by a mineral or organic acid or by a base in order to obtain the corresponding salt,
u) resolving of the racemic forms to resolved forms, said products as defined in claim 1 thus obtained being in all possible racemic, enantiomeric and diastereoisomeric isomer forms.

7. As medicaments, the products as defined in claim 5, these products being in all possible racemic, enantiomeric and diastereoisomeric isomer forms, as well as the addition salts with pharmaceutically acceptable mineral and organic acids or with mineral and organic bases of these products as defined in claim 1.

8. Pharmaceutical compositions containing as active ingredient, at least one of the medicaments as defined in claim 7 .

## Claims

1. Verwendung von Verbindungen der Formel (I) 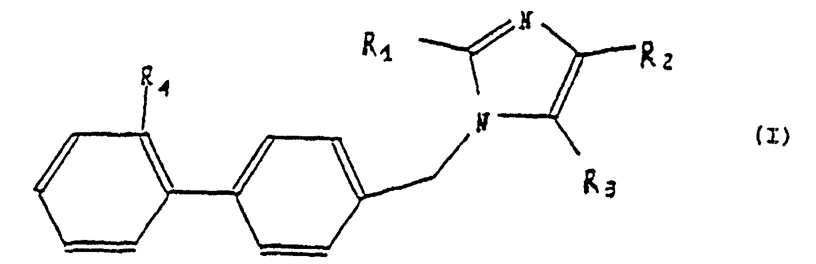 entsprechend den folgenden Formeln:
2-Butyl-α-hydroxy-α-methyl-4-(methylthio)-1-[(2'-((((phenylmethoxy)carbonyl)amino)sulfonyl)-(1,1'-biphenyl)-4-yl)methyl]-1H-imidazol-5-essigsäure,
2-Butyl-α-hydroxy-α-methyl-4-(methylthio)-1-[(2'-((((propylamino)carbonyl)amino)sulfonyl)-(1,1'-biphenyl)-4-yl)-methyl]-1H-imidazol-5-essigsäure,
2-Butyl-α-hydroxy-α-methyl-4-(methylthio)-1-[(2'-((((phenylmethyl)amino)carbonyl)amino)sulfonyl)-(1,1'-biphenyl)-4-yl)methyl]-1H-imidazol-5-essigsäure,
2-Butyl-1-[(2'-(((((cyclohexylmethyl)amino)carbonyl)-amino)sulfonyl)-(1,1'-biphenyl)-4-yl)methyl]-α-hydroxy-α-methyl-4-(methylthio)-1H-imidazol-5-essigsäure,
1-[(2'-(((phenylmethyl)amino)sulfonyl)-(1,1'-biphenyl)-4-yl)methyl]-α-hydroxy-4-(methylthio)-2-propyl-1H-imid-azol-5-essigsäure,
1-[(2'-(((((Cyclohexylmethyl)amino)carbonyl)amino)sulfonyl)-(1,1'-biphenyl)-4-yl)methyl]-4-(methylthio)-2-propyl-1H-imidazol-5-natriumcarboxylat,
4-(Methylthio)-1-[(2'-(((((phenylmethyl)amino)carbonyl)-amino)sulfonyl)-(1,1'-biphenyl)-4-yl)methyl]-2-propyl-1H-imidazol-5-carbonsäure,
α-Butyl-α-hydroxy-4-(methylthio)-1-[(2'-(((((phenylmethyl)amino)carbonyl)amino)sulfonyl)-(1,1'-biphenyl)-4-yl)methyl]-2-propyl-1H-imidazol-5-natriumacetat,
α-Hydroxy-4-(methylthio)-α-phenyl-1-[(2'-(((((phenylmethyl)amino)carbonyl)amino)sulfonyl)-(1,1'-biphenyl)-4-yl)methyl]-2-propyl-1H-imidazol-5-essigsäure,
wobei die Verbindungen der Formel (I) in allen möglichen isomeren racemischen, enantiomeren und diastereoisomeren Formen vorliegen, sowie der Additionssalze mit mineralischen und organischen Säuren oder mit mineralischen und organischen Basen der Verbindungen der Formel (I) zur Herstellung von pharmazeutischen Zusammensetzungen zur Behandlung von Störungen, die das Ergebnis einer anormalen Stimulation der AT₁- und AT₂-Angiotensin-II-Rezeptoren sind.

2. Verwendung der Verbindungen wie in Anspruch 1 definiert, zur Herstellung von pharmazeutischen Zusammensetzungen zur Behandlung von arterieller Hypertension, Herzinsuffizienz und post-angioplastischen Restenosen.

3. Verwendung der Verbindungen wie in Anspruch 1 definiert, zur Herstellung von pharmazeutischen Zusammensetzungen zur Behandlung von Niereninsuffizienz.

4. Verwendung der Verbindungen wie in Anspruch 1 definiert, zur Herstellung von pharmazeutischen Zusammensetzungen zur Behandlung und zur Verhütung von kardiovaskulären Störungen im Zusammenhang mit Diabetes.

5. Verbindungen der folgenden Formeln:
2-Butyl-α-hydroxy-α-methyl-4-(methylthio)-1-[(2'-((((phenylmethoxy)carbonyl)amino)sulfonyl)-(1,1'-biphenyl)-4-yl)methyl]-1H-imidazol-5-essigsäure,
2-Butyl-α-hydroxy-α-methyl-4-(methylthio)-1-[(2'-((((propylamino)carbonyl)amino)sulfonyl)-(1,1'-biphenyl)-4-yl)-methyl]-1H-imidazol-5-essigsäure,
2-Butyl-α-hydroxy-α-methyl-4-(methylthio)-1-[(2'-((((phenylmethyl)amino)carbonyl)amino)sulfonyl)-(1,1'-biphenyl)-4-yl)methyl]-1H-imidazol-5-essigsäure,
2-Butyl-1-[(2'-(((((cyclohexylmethyl)amino)carbonyl)-amino)sulfonyl)-(1,1'-biphenyl)-4-yl)methyl]-α-hydroxy-α-methyl-4-(methylthio)-1H-imidazol-5-essigsäure,
1-[(2'- (((phenylmethyl)amino)sulfonyl)-(1,1'-biphenyl)-4-yl)methyl]-α-hydroxy-4-(methylthio)-2-propyl-1H-imidazol-5-essigsäure,
1-[(2'-(((((Cyclohexylmethyl)amino)carbonyl)amino)sulfonyl)-(1,1'-biphenyl)-4-yl)methyl]-4-(methylthio)-2-propyl-1H-imidazol-5-natriumcarboxylat,
4-(Methylthio)-1-[(2'-(((((phenylmethyl)amino)carbonyl)-amino)sulfonyl)-(1,1'-biphenyl)-4-yl)methyl]-2-propyl-1H-imidazol-5-carbonsäure,
α-Butyl-α-hydroxy-4-(methylthio)-1-[(2'-(((((phenylmethyl)amino)carbonyl)amino)sulfonyl)-(1,1'-biphenyl)-4-yl)methyl]-2-propyl-1H-imidazol-5-natriumacetat,
α-Hydroxy-4-(methylthio)-α-phenyl-1-[(2'-(((((phenylmethyl)amino)carbonyl)amino)sulfonyl)-(1,1'-biphenyl)-4-yl)methyl]-2-propyl-1H-imidazol-5-essigsäure,

6. Verfahren zur Herstellung von Verbindungen wie in Anspruch 1 definiert, **dadurch gekennzeichnet, dass** man
entweder eine Verbindung der Formel (II): 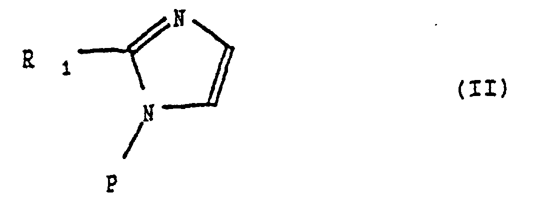 worin R₁ wie in Anspruch 1 definiert ist und P eine Schutzgruppe des Stickstoffatoms bedeutet,
einer Halogenierungsreaktion unterwirft, um eine Verbindung der Formel (III) zu erhalten: 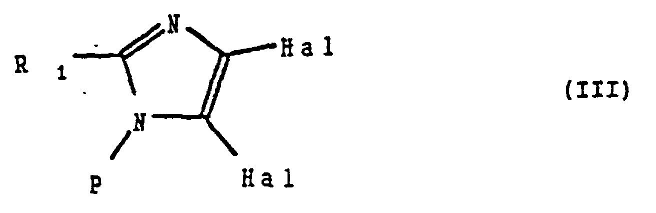 worin R₁ und P wie vorstehend definiert sind und Hal ein Halogenatom bedeutet, welche man einer Halogen-Metall-Austauschreaktion an einem der Halogenatome unterwirft, dann mit einer Verbindung der Formel (IV_{c}) oder (IV_{d}): 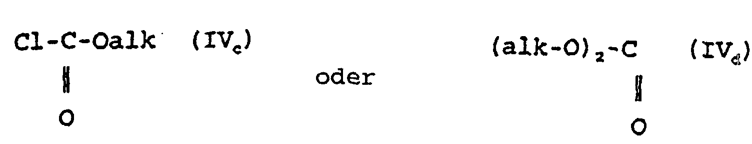 worin alk einen Alkylrest, umfassend höchstens 4 Kohlenstoff- atome, darstellt, umsetzt, um die Verbindung der Formal (V): 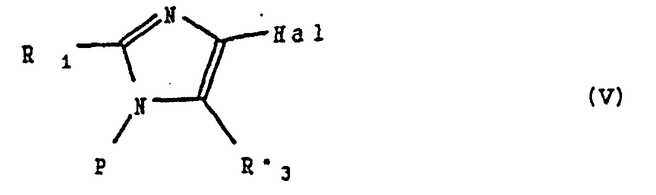 zu erhalten, worin R₁, P und Hal wie vorstehend definiert sind und R"₃ CO-O-alk, wie vorstehend definiert, bezeichnet,
wobei man die Verbindung der Formel (V) einer Halogen-Metall-Austauschreaktion an einem Halogenatom unterwirft und dann mit einer Verbindung der Formel (IVₐ) oder (Iv_{b})
(-S-R')₂ (Ivₐ) oder MeSO₂SR' (Iv_{b})
umsetzt, worin SR' die Bedeutung von R₂ gemäß Anspruch 1 besitzt, um die Verbindung der Formel (VII): 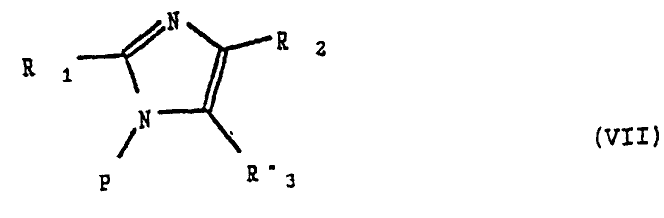 zu erhalten, worin R₁, R₂, R"₃ und P wie vorstehend definiert sind, wobei man bei der Verbindung der Formel (VII), die durch P, wie vorstehend definiert, blockierte Aminfunktion freisetzt, dann mit einer Verbindung der Formel (VIII) : 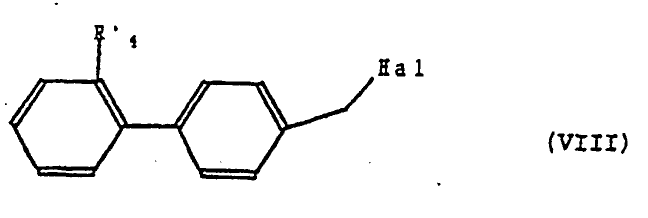 umsetzt, worin R'₄ wie in Anspruch 1 für R₄ definiert ist, worin die möglichen reaktiven Funktionen gegebenenfalls durch Schutzgruppen geschützt sind und Hal ein Halogenatom bedeutet, um eine Verbindung der Formel (I₁): 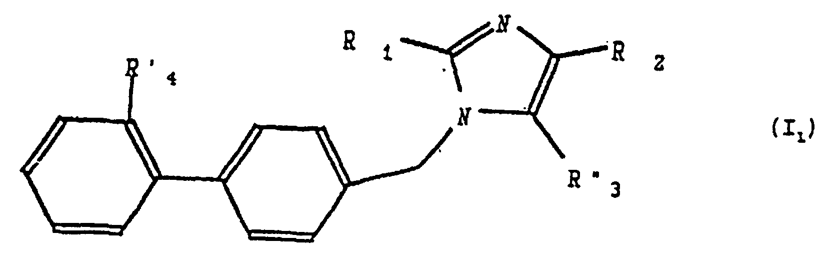 zu erhalten, worin R₁, R₂, R"₃ und R'₄ wie vorstehend definiert sind,
oder man eine Verbindung der Formel (IX): 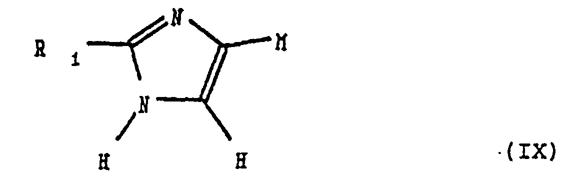 worin R₁ wie vorstehend definiert ist und M ein Wasserstoffatom oder den Rest R₂, der die vorstehend angegebene Bedeutung hat, bezeichnet, mit der Verbindung der Formel (VIII), wie vorstehend definiert, umsetzt, um eine Verbindung der Formel (X): 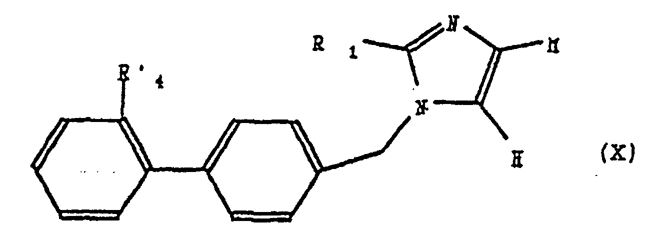 zu erhalten, worin R₁, M und R'₄ wie vorstehend definiert sind, wobei man die Verbindung der Formel (X), wenn M ein Wasserstoffatom bedeutet, einer Halogenierungsreaktion unterwirft, um die Verbindung der Formel (XIV): 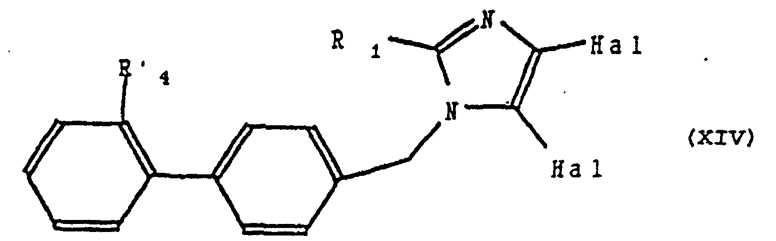 zu erhalten, worin R₁, R'₄ und Hal wie vorstehend definiert sind, die man einer Halogen-Metall-Austauschreaktion unterwirft, dann der Wirkung einer Verbindung der Formal (IV_{c}) oder (IV_{d}), wie vorstehend definiert, unterwirft, um die Verbindung der Formel (XXI): 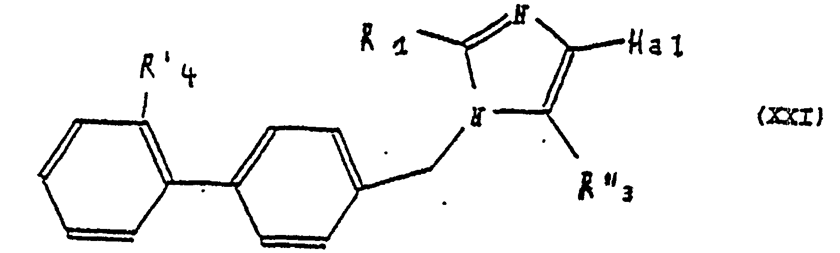 zu erhalten, worin R₁, R'₄, Hal und R"₃ wie vorstehend definiert sind, wobei die Verbindung der Formel (XXI) einer Halogen-Metall-Austauschreaktion unterworfen wird, dann mit einer Verbindung der Formel (IVₐ) oder (IV_{b}), wie vorstehend definiert, umgesetzt wird, um eine Verbindung der Formel (I₁) : 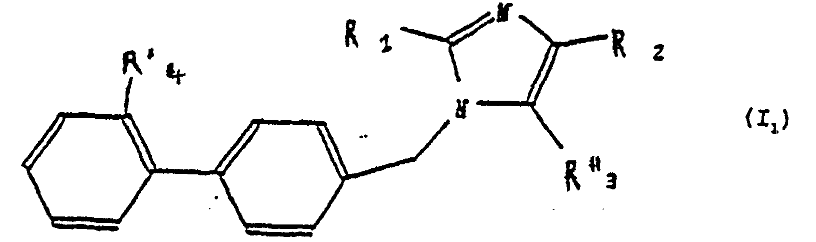 zu erhalten, worin R₁, R'₄, R₂ und R"₃ wie vorstehend definiert sind,
oder man eine Verbindung der Formel (XX): 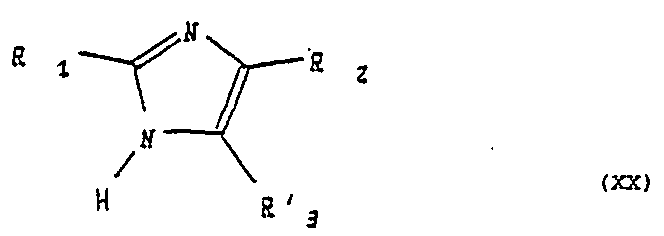 worin R₁ und R₂ wie vorstehend definiert sind und R'₃ wie in Anspruch 1 für R₃ definiert ist, worin die möglichen reaktiven Funktionen gegebenenfalls durch Schutzgruppen geschützt sind, einer Reaction mit der Verbindung der Formel (VIII), wie vorstehend definiert, unterwirft, um eine Verbindung der Formel (I'): 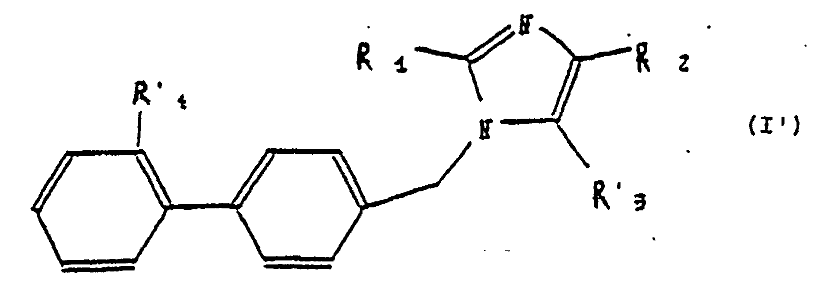 zu erhalten, worin R₁, R₂, R'₃ und R'₄ wie vorstehend definiert sind,
wobei man die Verbindungen der Formel (I'), die Verbindungen der Formel (I) sein können und die man zum Erhalt dieser oder anderer Verbindungen der Formel (I) gegebenenfalls und sofern notwendig, sowie die Verbindungen der Formel (I₁) einer oder mehreren der folgenden Transformationsreaktionen in einer beliebigen Reihenfolge unterwirft:
b) Verseifung der Esterfunktion in eine Säurefunktion,
d) Transformation der Cyanofunktion in eine Säurefunktion,
e) Transformation der Säurefunktion in eine Amidfunktion,
g) Transformation der Alkoxyfunktion in eine Hydroxylfunktion,
h) Oxidation der Alkoholfunktion in eine Säurefunktion,
i) Transformation eines Formylrests in einen Carbamoylrest,
r) Transformation eines Carbamats in Harnstoff und insbesondere eines Sulfonylcarbamats in Sulfonylharnstoff,
s) Abspaltung der Schutzgruppen, die die reaktiven geschützten Funktionen tragen können,
t) Verseifung durch eine mineralische oder organische Säure oder durch eine Base zum Erhalt des entsprechenden Salzes,
u) Aufspaltung der racemischen Formen in die getrennten Verbindungen, wobei die Verbindungen wie in Anspruch 1 definiert sind und auch in allen möglichen isomeren racemischen, enantiomeren und diastereoisomeren Formen vorliegen.

7. Als Medikamente Verbindungen, wie in Anspruch 5 definiert, wobei diese Verbindungen in allen möglichen isomeren racemischen, enantiomeren und diastereoisomeren Formen vorliegen, sowie die Additionssalze mit den mineralischen und organischen pharmazeutisch verträglichen Säuren oder mit den mineralischen und organischen pharmazeutisch verträglichen Basen dieser Verbindungen, wie in Anspruch 1 definiert.

8. Pharmazeutische Zusammensetzungen, die als Wirkstoff mindestens eines der Medikamente wie in Anspruch 7 definiert enthalten.
